(19)

Europäisches
Patentamt

European
Patent Office

Office européen
des brevets

(11) **EP 4 163 916 B1**

(12) **EUROPEAN PATENT SPECIFICATION**

(45) Date of publication and mention
of the grant of the patent:
**20.08.2025 Bulletin 2025/34**

(21) Application number: **22170687.2**

(22) Date of filing: **29.04.2022**

(51) International Patent Classification (IPC):
**G16C 20/30** $^{(2019.01)}$    **G06N 3/00** $^{(2023.01)}$

(52) Cooperative Patent Classification (CPC):
**G16C 20/30; G06N 3/042; G06N 3/0442;**
**G06N 3/0455; G06N 3/0464; G06N 3/084;**
**G06N 3/09;** G16C 20/70

(54) **SYSTEM AND METHOD FOR MOLECULAR PROPERTY PREDICTION USING HIERARCHICAL LAYER-WISE PROPAGATION OF GRAPH POOLING LAYER**

SYSTEM UND VERFAHREN ZUR VORHERSAGE MOLEKULARER EIGENSCHAFTEN UNTER VERWENDUNG EINER HIERARCHISCHEN SCHICHTWEISEN AUSBREITUNG EINER GRAPHEN-POOLING-SCHICHT

SYSTÈME ET PROCÉDÉ DE PRÉDICTION DE PROPRIÉTÉS MOLÉCULAIRES À L'AIDE D'UNE PROPAGATION HIÉRARCHIQUE PAR COUCHES D'UNE COUCHE DE REGROUPEMENT DE GRAPHES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.10.2021 IN 202121046301**

(43) Date of publication of application:
**12.04.2023 Bulletin 2023/15**

(73) Proprietor: **Tata Consultancy Services Limited Maharashtra (IN)**

(72) Inventors:
• **SAKHINANA, Sagar Srinivas**
**411013 Pune (IN)**
• **BUDDHIRAJU, Venkata Sudheendra**
**411013 Pune (IN)**
• **NISTALA, Sri Harsha**
**411013 Pune (IN)**
• **RUNKANA, Venkataramana**
**411013 Pune (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• **JUSTIN GILMER ET AL: "Neural Message Passing for Quantum Chemistry", COMPUTER SCIENCE, 4 April 2017 (2017-04-04), pages 1 - 14, XP055700744, Retrieved from the Internet <URL:https://arxiv.org/abs/1704.01212>**
• **GAO HONGYANG ET AL: "Graph U-Nets", IEEE TRANSACTIONS ON PATTERN ANALYSIS AND MACHINE INTELLIGENCE, IEEE COMPUTER SOCIETY, USA, vol. 44, no. 9, 14 May 2021 (2021-05-14), pages 4948 - 4960, XP011916156, ISSN: 0162-8828, [retrieved on 20210517], DOI: 10.1109/TPAMI.2021.3081010**

EP 4 163 916 B1

**Description**

CROSS-REFERENCE TO RELATED APPLICATIONS AND PRIORITY

The present application claims priority to Indian application, Application No. 202121046301, filed in India on October 11, 2021.

TECHNICAL FIELD

The disclosure herein generally relates to field of molecular property prediction, and, more particularly, to system and method for molecular property prediction using hierarchical layer-wise propagation of graph pooling layer.

BACKGROUND

Machine learning technique, in specific, supervised learning on drug-like potential molecules has remarkable applications for use in more effective drug discovery. It provides substantial prospects in diminishing the computational complexity which is a key desideratum for prognostication of molecular properties and aid in billion price tag cost reduction of developing a potential drug for treatment.

Message Passing Neural Networks (MPNN's) are non-spectral approach of performing convolution on unstructured molecular graphs. It is a graph-based property prediction framework. It leverages a message passing algorithm and a set-pooling aggregation operator to derive a graph-level representation of the complete input low treewidth chemical graphs to assist in inductive learning tasks. The MPNN's however suffer from inherent limitation to effectively encapsulate the characteristics of the molecular graph. Moreover, due to its high computational complexity, MPNN in not viable for real-time property prediction. Gilmer, J. et al. ("Neural Message Passing for Quantum Chemistry", Computer Science, 4 April 2017, pages 1-14) discloses message passing neural networks for the prediction of molecular properties.

SUMMARY

The invention is defined in the appended claims. Embodiments of the present disclosure present technological improvements as solutions to one or more of the above-mentioned technical problems recognized by the inventors in conventional systems. For example, in one embodiment, a processor implemented method for molecular property prediction is provided. The method includes accessing, via one or more hardware processors, a database comprising a plurality of molecular graphs associated with a plurality of molecules and a plurality of labels indicative of chemical properties of the plurality of the molecular graphs, wherein each molecular graph of the plurality of molecular graphs comprises a plurality of nodes and a plurality of edges connecting a plurality of neighboring nodes. Further the method includes performing, via the one or more hardware processors, a first iteration to down-sample the molecular graph into a coarsened molecular graph. The first iteration includes obtaining a real-valued feature matrix of a molecular graph from amongst the plurality of molecular graphs, each row vector of the real-valued feature matrix represents a feature attribute of the plurality of nodes of the molecular graph; transforming feature attribute $\mathbf{f}_i^{\ell}$ associated with the node by taking a product of the feature attribute $\mathbf{f}_i^{\ell}$ with a feed-forward layer

$$\Gamma_{\Theta}\left(\mathbf{e}_{j,i}^{\mathcal{M}}\right),$$

to obtain edge-information aware node attributes

$$\mathbf{f}_i^{\ell} \; \Gamma_{\Theta}\left(\mathbf{e}_{j,i}^{\mathcal{M}}\right),$$

wherein the feed-forward layer comprises a neural network function, parameterized by $\Theta$; computing a scalar projection $\mathbf{z}_i$ of the real-valued feature matrix $F^{\ell}$ on a projection vector $\mathbf{v}^{\ell}$, the scalar projection $\mathbf{z}_i$ comprises a projected scalar value of each node-attribute in the molecular graph on to the projection vector $\mathbf{v}^{\ell}$, and wherein the scalar projection $\mathbf{z}_i$ further measures a feature information of the node i to be retained when projected in the direction of learnable vector $\mathbf{v}^{\ell}$; obtaining a hierarchical layer-wise propagation of a graph pooling layer of the molecular graph by taking a product of the edge-information aware node attributes

$$\mathbf{f}_i^{\ell} \; \Gamma_{\Theta}(\mathbf{e}_{j,i}^{\mathcal{M}})$$

and a unit vector associated with the projection vector $\mathbf{v}^{\ell}$; down-sampling the molecular graph using the hierarchical layer-wise propagation of the graph pooling layer, wherein the down-sampling of the molecular graph comprises performing a m-max-pooling operation on the molecular graph to sample a subset of m top-ranked nodes to form a coarsened molecular graph, wherein the down-sampling of the molecular graph results in rejecting a first set of nodes and retaining a second set of nodes from amongst a plurality of nodes of the molecular graph based on a ranking of the plurality of nodes, and wherein the ranking of the plurality of nodes is performed by utilizing a scalar projection scores to sample indexes of the second set of nodes; determining a first adjacency matrix of the coarsened molecular graph using the second set of nodes; determining a first feature matrix of the coarsened molecular graph using the second set of nodes, wherein each row of the first feature matrix corresponds to hidden state node attributes of the coarsened molecular graph. Further, the method includes performing, via the one or more hardware processors, one or more second iterations, wherein each of the one or more second iterations comprises performing, on the coarsened molecular graph of an immediately preceding iteration of the one or more second iterations, obtaining a real-valued feature matrix of the coarsened molecular graph, transforming feature attribute of the node, computing the scalar projection $\mathbf{z_i}$ of the real-valued feature matrix, obtaining the hierarchical layer-wise propagation of a graph pooling layer of the coarsened molecular graph, down-sampling the coarsened molecular graph using the hierarchical layer-wise propagation of the graph pooling layer, and determining a second adjacency matrix and a second feature matrix of the coarsened molecular graph. Furthermore, the method includes computing, via the one or more hardware processors, an average of the hidden state node attributes of the coarsened molecular graph obtained after preforming the one or more second iterations to obtain a graph level representation vector of the molecular graph. Also, the method includes determining, via the one or more hardware processors, one or more molecular properties using a linear layer from the graph level representation vector.

In another aspect, a system for molecular property prediction is provided. The system includes a memory storing instructions; one or more communication interfaces; and one or more hardware processors coupled to the memory via the one or more communication interfaces, wherein the one or more hardware processors are configured by the instructions to access a database comprising a plurality of molecular graphs associated with a plurality of molecules and a plurality of labels indicative of chemical properties of the plurality of the molecular graphs, wherein each molecular graph of the plurality of molecular graphs comprises a plurality of nodes and a plurality of edges connecting a plurality of neighboring nodes. Further, the one or more hardware processors are configured by the instructions to perform a first iteration to down-sample the molecular graph into a coarsened molecular graph, wherein to perform the first iteration the one or more hardware processors are configured by the instructions to obtain a real-valued feature matrix of a molecular graph from amongst the plurality of molecular graphs, each row vector of the real-valued feature matrix represents a feature attribute of the plurality of nodes of the molecular graph; transform feature attribute $\mathbf{f}_i^{\ell}$ associated with the node by taking a product of the feature attribute $\mathbf{f}_i^{\ell}$ with a feed-forward layer

$$\Gamma_{\Theta}(\mathbf{e}_{j,i}^{\mathcal{M}}),$$

to obtain edge-information aware node attributes

$$\mathbf{f}_i^{\ell} \; \Gamma_{\Theta}(\mathbf{e}_{j,i}^{\mathcal{M}}),$$

wherein the feed-forward layer comprises a neural network function, parameterized by $\Theta$; compute a scalar projection $\mathbf{z_i}$ of the real-valued feature matrix $F^{\ell}$ on a projection vector $\mathbf{v}^{\ell}$, the scalar projection $\mathbf{z_i}$ comprises a projected scalar value of each node-attribute in the molecular graph on to the projection vector $\mathbf{v}^{\ell}$, and wherein the scalar projection $\mathbf{z_i}$ further measures a feature information of the node i to be retained when projected in the direction of learnable vector $\mathbf{v}^{\ell}$; obtain a hierarchical layer-wise propagation of a graph pooling layer of the molecular graph by taking a product of the edge-information aware node attributes

$$\mathbf{f}_i^{\ell} \; \Gamma_{\Theta}(\mathbf{e}_{j,i}^{\mathcal{M}})$$

and a unit vector associated with the projection vector $\mathbf{v}^{\ell}$; down-sample the molecular graph using the hierarchical layer-wise propagation of the graph pooling layer, wherein the down-sampling of the molecular graph comprises performing a m-max-pooling operation on the molecular graph to sample a subset of m top-ranked nodes to form a coarsened molecular graph, wherein the down-sampling of the molecular graph results in rejecting a first set of nodes and retaining a second set of nodes from amongst a plurality of nodes of the molecular graph based on a ranking of the plurality of nodes, and wherein the ranking of the plurality of nodes is performed by utilizing a scalar projection scores to sample indexes of the second set of nodes; determine a first adjacency matrix of the coarsened molecular graph using the second set of nodes; and determine a first feature matrix of the coarsened molecular graph using the second set of nodes, wherein each row of the first feature matrix corresponds to hidden state node attributes of the coarsened molecular graph; perform one or more second iterations, wherein each of the one or more second iterations comprises performing, on the coarsened molecular graph of an immediately preceding iteration of the one or more second iterations, obtaining a real-valued feature matrix of the coarsened molecular graph, transforming feature attribute of the node, computing the scalar projection $z_i$ of the real-valued feature matrix, obtaining the hierarchical layer-wise propagation of a graph pooling layer of the coarsened molecular graph, down-sampling the coarsened molecular graph using the hierarchical layer-wise propagation of the graph pooling layer, and determining a second adjacency matrix and a second feature matrix of the coarsened molecular graph. Furthermore, the one or more hardware processors are configured by the instructions to compute an average of the hidden state node attributes of the coarsened molecular graph obtained after preforming the one or more second iterations to obtain a graph level representation vector of the molecular graph. Moreover, the one or more hardware processors are configured by the instructions to determine one or more molecular properties using a linear layer from the graph level representation vector.

In yet another aspect, a non-transitory computer readable medium for a method for molecular property prediction is provided. The non-transitory computer readable medium includes a plurality of instructions, which when executed cause molecular property prediction via the following method steps. The method steps includes accessing a database comprising a plurality of molecular graphs associated with a plurality of molecules and a plurality of labels indicative of chemical properties of the plurality of the molecular graphs, wherein each molecular graph of the plurality of molecular graphs comprises a plurality of nodes and a plurality of edges connecting a plurality of neighboring nodes. Further the method steps include performing a first iteration to down-sample the molecular graph into a coarsened molecular graph. The first iteration includes obtaining a real-valued feature matrix of a molecular graph from amongst the plurality of molecular graphs, each row vector of the real-valued feature matrix represents a feature attribute of the plurality of nodes of the molecular graph; transforming feature attribute $\mathbf{f}_i^\ell$ associated with the node by taking a product of the feature attribute $\mathbf{f}_i^\ell$ with a feed-forward layer

$$\Gamma_\Theta(\mathbf{e}_{j,i}^{\mathcal{M}}),$$

to obtain edge-information aware node attributes

$$\mathbf{f}_i^\ell \ \Gamma_\Theta(\mathbf{e}_{j,i}^{\mathcal{M}}),$$

wherein the feed-forward layer comprises a neural network function, parameterized by $\Theta$; computing a scalar projection $z_i$ of the real-valued feature matrix $F^\ell$ on a projection vector $\mathbf{v}^\ell$, the scalar projection $z_i$ comprises a projected scalar value of each node-attribute in the molecular graph on to the projection vector $\mathbf{v}^\ell$, and wherein the scalar projection $z_i$ further measures a feature information of the node i to be retained when projected in the direction of learnable vector $\mathbf{v}^\ell$; obtaining a hierarchical layer-wise propagation of a graph pooling layer of the molecular graph by taking a product of the edge-information aware node attributes

$$\mathbf{f}_i^\ell \ \Gamma_\Theta(\mathbf{e}_{j,i}^{\mathcal{M}})$$

and a unit vector associated with the projection vector $\mathbf{v}^\ell$; down-sampling the molecular graph using the hierarchical layer-wise propagation of the graph pooling layer, wherein the down-sampling of the molecular graph comprises performing a m-max-pooling operation on the molecular graph to sample a subset of m top-ranked nodes to form a coarsened molecular graph, wherein the down-sampling of the molecular graph results in rejecting a first set of nodes and retaining a second set of nodes from amongst a plurality of nodes of the molecular graph based on a ranking of the plurality of nodes, and wherein the ranking of the plurality of nodes is performed by utilizing a scalar projection scores to sample indexes of the second set of nodes; determining a first adjacency matrix of the coarsened molecular graph using the second set of nodes;

determining a first feature matrix of the coarsened molecular graph using the second set of nodes, wherein each row of the first feature matrix corresponds to hidden state node attributes of the coarsened molecular graph. Further, the method steps includes performing one or more second iterations, wherein each of the one or more second iterations comprises performing, on the coarsened molecular graph of an immediately preceding iteration of the one or more second iterations, obtaining a real-valued feature matrix of the coarsened molecular graph, transforming feature attribute of the node, computing the scalar projection $z_i$ of the real-valued feature matrix, obtaining the hierarchical layer-wise propagation of a graph pooling layer of the coarsened molecular graph, down-sampling the coarsened molecular graph using the hierarchical layer-wise propagation of the graph pooling layer, and determining a second adjacency matrix and a second feature matrix of the coarsened molecular graph. Furthermore, the method steps includes computing an average of the hidden state node attributes of the coarsened molecular graph obtained after preforming the one or more second iterations to obtain a graph level representation vector of the molecular graph. Also, the method steps include determining one or more molecular properties using a linear layer from the graph level representation vector.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A illustrates a representation of message passing phase of a conventional message passing neural network (MPNN).
FIG. 1B illustrates a representation of readout phase of a conventional message passing neural network (MPNN).
FIG. 2 illustrates a network implementation of a system for molecular property prediction using hierarchical layer-wise propagation of graph pooling layer according to some embodiments of the present disclosure.
FIGS. 3A and 3B is a flow diagram illustrating a method for molecular property prediction using hierarchical layer-wise propagation of graph pooling layer in accordance with some embodiments of the present disclosure.
FIG. 4 illustrates an example representation of down-sampling of a molecular graph and upsampling of a resultant coarsened molecular graph by leveraging a gPool layer in an encoder block, in accordance with an example embodiment.
FIG. 5 is a block diagram of an exemplary computer system for implementing embodiments consistent with the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Convolution Neural Networks (CNNs) have contributed to a broad range of scientific breakthroughs, in the fields as deep residual learning for visual recognition, self-driving cars, optical character recognition engine, acoustic modeling in speech recognition, neural machine translation, etc. In view of the inherent methodology, CNN and its variant structured neural network architectures obtain dimensionality reduction and extract dominant features by performing spatial convolutions on Euclidean domains. The ConvNets input is obliged to, therefore, have a regular and grid-like structure. This restriction hampers the utilization of CNN's to numerous scientific disciplines, where irregular graph structure or manifold data are commonly accessible.

Chemical graph theory treats drug-like organic molecules as non-linear static graphs. Message Passing Neural Networks (MPNN) framework merges several distinct and unique contemporary models that exist in the literature. The MPNNs are an abstraction of a non-spectral approach based Graph Convolution Networks. The MPNNs operate on undirected chemical graphs, $\mathcal{G}^{\mathcal{M}} = (\mathcal{V}^{\mathcal{M}}, \mathcal{E}^{\mathcal{M}})$.

Let $\mathcal{N}^{\mathcal{M}}(|\mathcal{V}^{\mathcal{M}}|)$ denote the number of nodes and $E^{\mathcal{M}}(|\mathcal{E}^{\mathcal{M}}|)$ represents the number of edges for a homogenous chemical graph, $\mathcal{G}^{\mathcal{M}}$. $\mathcal{G}^{\mathcal{M}}$ is described by a set of node features,

$$N^{\mathcal{M}} \in R^{|V^{\mathbf{M}}| \times \mathbb{C}}, N_i^{\mathcal{M}} \in R^{1 \times \mathbb{C}} \forall i \in \mathcal{V}^{\mathcal{M}}$$

and edge features

$$e_{ij}^{\mathcal{M}} \in R^{|E^{\mathbf{M}}| \times \mathbb{Z}}, \forall(i,j) \in \mathcal{E}^{\mathcal{M}}.$$

Here, $i \& j \in \mathcal{V}^{\mathcal{M}}$ refer to the neighboring nodes of the chemical graph and are connected by an arbitrary edge, $(i, j) \in \mathcal{E}^{\mathcal{M}}$ $\leftrightarrow (j, i) \in \mathcal{E}^{\mathcal{M}}, \forall j \in \mathcal{N}(i)$. The chemical graph connectivity

$$\mathcal{G}^{\mathcal{M}} = (\mathcal{V}^{\mathcal{M}}, \mathcal{E}^{\mathcal{M}})$$

is given by the adjacency matrix,

$$\mathcal{G}_{\mathcal{A}}^{\mathcal{M}}.$$

Molecules are represented as annotated undirected chemical graphs. The atoms are considered to be the nodes of the chemical graphs. The bonds connecting adjacent atoms in the chemical graphs correspond to edges. The MPNN framework is leveraged in this particular scientific discipline for mathematical modeling of the drug-like organic molecules. It helps to gain insight and assists in the description of the chemical graph's topology into a characteristic representation of the entire chemical graphs to later aid in the molecular property prediction task. Based on the graph's connectivity, an incident edge in between two neighboring atoms in the chemical compound (or nodes in a chemical graph) acts as both arriving and departing edge tagged by the same label (bond type).

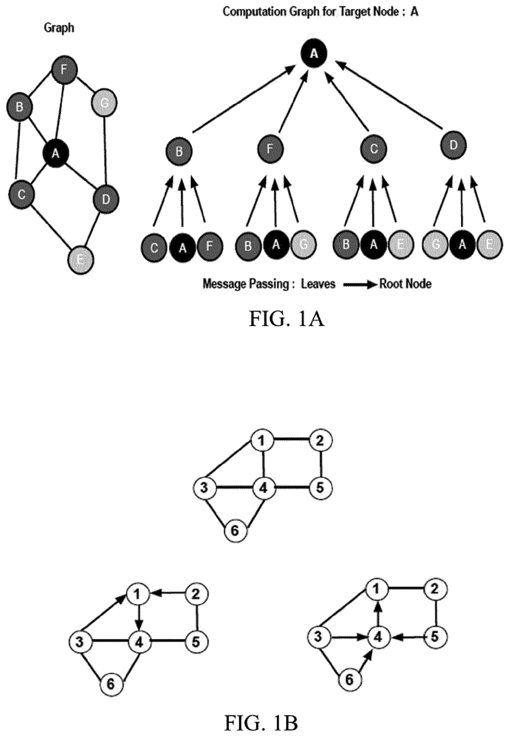

The MPNNs forward pass consists of two phases - a message passing phase (illustrated in FIG. 1A) and a readout phase (illustrated in FIG. 1B). The message passing phase generates neural messages and update node representations by aggregating encoded information of node's embeddings from confined graph neighborhood. A permutation invariant readout phase is leveraged to perform graph pooling. Readout phase function takes an adjacency matrix

$$\mathcal{G}_A^{\mathcal{M}}$$

as input and satisfies the following property,

$$\mathcal{F}\left(P\mathcal{G}_A^{\mathcal{M}}P^{\top}\right) = \mathcal{F}(\mathcal{G}_A^{\mathcal{M}}).$$

Here, P is a permutation matrix. The message propagation utilizes the distinct message generating functions acting on the undirected chemical graph topology $M_{in}^{f}$ and $M_{out}^{f}$ represents the universal function approximator for the generation of neural messages received through a particular edge type and propagated from the same edge-type between the nodes of the chemical graphs, respectively. Based on the direction of the edge under consideration, that particular transformation function is put into operation on the edge,

$$e_{ji}^{\mathcal{M}}.$$

The MPNNs forward pass communicates messages as described by a computational graph for every target node from the bottom (leaf) node to the top (root) node by iterating for $T$ computational time steps. These outgoing messages are evaluated for every edge by transforming the source node's hidden state according to the edge feature vector.

Identical edge-type incident on different node neighbors, which is characterized by distinct edge feature vectors share with the same instance of the message generating neural-network function. Each node in the molecular graph aggregates neural messages (message-pooling operation) from its local T-hop neighbors, as determined from

$$\mathcal{G}_A^{\mathcal{M}}$$

and the received messages are perceived by the target node by performing mathematical computations to update its hidden representation. This message-passing scheme assists in learning effective discriminative hidden representations of the nodes in the chemical graphs, by adopting a Back-propagation through time (BPTT) learning algorithm for training the MPNNs framework when solved for graph-based inductive learning tasks. The MPNNs are synchronous message-passing systems. They update all messages in parallel. In the end, each edge between the vertices in the chemical graph have messages evaluated in both directions from the source to sink and contrariwise. The message passing phase is described by utilizing a message generating function, $M^f$, and node-embedding update neural network function, $V^f$. $M^f$ and $V^f$ might take possession of diverse in specific to be at variance with function settings. During the span of the message passing phase, the node-level embedding $\mathbf{h}_i^t$ of every unique vertex in the molecular graph as given by its computational graph are overhauled and assembled on structural and feature information embedded messages $m_i^{t+1}$., received from its one-hop neighbors as depicted by,

$$m_i^{t+1} = \sum_{j \in N(i)} M^f\left(h_j^t, e_{ji}^{\mathcal{M}}\right) \tag{1}$$

$$h_i^{t+1} = V^f\left(h_i^t, m_i^{t+1}\right) \tag{2}$$

Here, $\sum_{i \in N(i)}$; depicts the aggregation of neural-information embedded messages over the local one-hop neighborhood of the node,

$$i \in \mathcal{V}^{\mathcal{M}}$$

in the chemical graph,

$$\mathcal{G}^{\mathcal{M}}.$$

Here, $h_i$ is learned with the MPNN model parameters through a representative function of the entire input graph when solved for addressing supervised graph regression tasks such as molecular graph property prediction. The readout phase of the MPNN framework performs graph-pooling through set-pooling approach by determining a discriminative graph embedding for the entire input molecular graph by utilizing a differentiable node-reordering invariant neural network function, $R^f$ according to,

$$\hat{y} = R^f\left(\{h_i^T | i \in \mathcal{V}^{\mathcal{M}}(\mathcal{G}^{\mathcal{M}})\}\right).$$

$M^f$, $V^f$, and $R^f$ are differentiable neural network functions and have learnable parameters. Mini-Batching with an MPNN with batch size as a hyper-parameter results in the faster training and augments performance of the graph-based deep learning algorithm. It is viable here in this context as it initiates and propagates neural messages across several molecular graphs with varying numbers of vertices $|\mathcal{V}^{\mathcal{M}}|$ and $|\mathcal{E}^{\mathcal{M}}|$. The feature representation of the vertices in the molecular graph,

$$\mathcal{G}^{\mathcal{M}}$$

is denoted by, *data.x. data.edgeindex* describes the edge indices of source and sink vertices of the edge under consideration and vice-versa. *data.edgeattr* represents the static edge attributes. *data.y* is the pre-determined DFT-evaluated properties (ground-truth) for the chemical graphs. The discrepancy between the MPNN model output (estimated) and the true values are measured by the mean-squared error loss function for this graph-based supervised regression task. The Edge-Conditioned Convolution Networks (ECCN) is described by,

$$\mathrm{h}_i^{t+1} = \Lambda \mathrm{h}_i^t + \sum_{j \in N(i)} \mathrm{h}_j^t \cdot \Omega_\Lambda(e_{ij}^{\mathcal{M}})$$

Here, $\Omega_\Lambda$ denotes a multilayer perceptron, parameterized by A. The aggregated vector message perceived by the sink node, i is described by

$$m_i^{t+1} = \sum_{j \in N(i)} \mathrm{h}_j^t \cdot \Omega_\Lambda(e_{ij}^{\mathcal{M}}).$$

The MPNN framework message-generating neural-network function is described by,

$$M^f\left(h_j^t, e_{ij}^{\mathcal{M}}\right) = \mathrm{h}_j^t \cdot \Omega_\Lambda\left(e_{ij}^{\mathcal{M}}\right).$$

The vertex update function is described by,

$$V^f\left(h_i^t, m_i^{t+1}\right): GRU\left(h_i^t, m_i^{t+1}\right).$$

Here, GRU is a known Gated Recurrent Unit. The hidden state of the previous state is given by,

$$V^f\left(h_i^t, m_i^{t+1}\right): GRU\left(h_i^t, m_i^{t+1}\right).$$

Here, n denotes the total number of nodes in the chemical graphs in a given batch size. $d_m$ & $d_h$ are the characteristic dimension of neural messages and node attributes respectively. Here, the reset gate, $R_t$, $Z_t$,

$$h_i^t, \tilde{h}_i^t$$

are evaluated as,

$$\mathrm{R}_t = \sigma\left(m_i^{t+1}\mathrm{W}_r + h_i^t\mathrm{W}_{rh} + \mathrm{b}_r\right), \qquad (3)$$

$$\mathrm{Z}_t = \sigma\left(m_i^{t+1}\mathrm{W}_z + h_i^t\mathrm{W}_{zh} + \mathrm{b}_z\right) \qquad (4)$$

$$\tilde{h}_i^t = \tanh(m_i^{t+1}\mathrm{W}_{\tilde{h}} + (\mathrm{R}_t \odot h_i^t)\mathrm{W}_{hh} + \mathrm{b}_h) \qquad (5)$$

$$h_i^{t+1} = \mathrm{Z}_t \odot h_i^t + (1 - \mathrm{Z}_t) \odot \tilde{h}_i^t \qquad (6)$$

Here,

$$\mathrm{W}_r, \mathrm{W}_z, \mathrm{W}_{\tilde{h}} \in \mathbb{R}^{d_m \times d_h}, \mathrm{W}_{rh}, \mathrm{W}_{zh}, \mathrm{W}_{hh} \in \mathbb{R}^{d_h \times d_h}$$

are the weight parameters, $\mathrm{b}_r, \mathrm{b}_z, \mathrm{b}_h \in \mathbb{R}^{1 \times d_h}$ are the bias.

The graph-level global pooling neural network is evaluated as

$$\hat{y} = R^f(\{h_i^T | i \in \mathcal{V}^{\mathcal{M}}(\mathcal{G}^{\mathcal{M}})\}) : Set2Set(\{h_i^T | i \in \mathcal{V}^{\mathcal{M}}(\mathcal{G}^{\mathcal{M}})\}) \quad (7)$$

The MPNN however leads to over-smoothing of learnable embeddings for vertices with higher valency. In addition, the MPNN's suffer from inherent limitation to effectively encapsulate the characteristics of the molecular graph. Moreover, due to its high computational complexity, the MPNNs are not viable for real-time molecular property prediction. Various embodiments described herein provide a method and system for molecular property prediction using an Edge-Conditioned Hierarchical Graph Pooling Layer. The disclosed system is resilient to noise by learning to adapt to kingpin on the task-relevant fragment of the molecular graphs at varying receptive fields, locality, and depth to augment the discriminative power of node and graph-level embeddings. The disclosed method learns the expressive/discriminative node and graph level embeddings to aid in molecular property prediction with a reduced computational complexity.

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

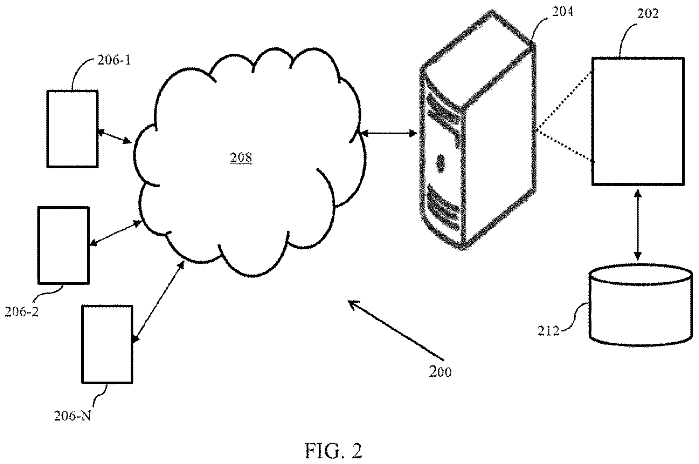

Referring now to the drawings, and more particularly to FIG. 2 through 5, where similar reference characters denote corresponding features consistently throughout the figures, there are shown preferred embodiments and these embodiments are described in the context of the following exemplary system and/or method.

FIG. 2 illustrates a network implementation 200 of a system for molecular property prediction according to some embodiments of the present disclosure. The disclosed system provides a supervised learning on drug-like potential molecules for use in effective drug discovery. It provides substantial prospects in diminishing the computational complexity which is a key desideratum for prognostication of molecular properties and aid in billion price tag cost reduction of developing a potential drug for treatment.

The proposed system comprises of message-passing, vertex update and graph-pooling modules which augment the graph-level prediction tasks accuracy by taking into account the structure of the graph. The proposed novel design of message-passing pipeline augments the graph-level prediction tasks accuracy by taking into account the structure of the graph.

Although the present disclosure is explained considering that the system 202 is implemented on a server, it may be understood that the system 202 may also be implemented in a variety of computing systems 204, such as a laptop computer, a desktop computer, a notebook, a workstation, a cloud-based computing environment and the like. It will be understood that the system 202 may be accessed through one or more devices 206-1, 206-2... 206-N, collectively referred to as devices 206 hereinafter, or applications residing on the devices 206. Examples of the devices 206 may include, but are not limited to, a portable computer, a personal digital assistant, a handheld device, a smartphone, a tablet computer, a workstation and the like. The devices 206 are communicatively coupled to the system 202 through a network 208.

In an embodiment, the network 208 may be a wireless or a wired network, or a combination thereof. In an example, the network 208 can be implemented as a computer network, as one of the different types of networks, such as virtual private network (VPN), intranet, local area network (LAN), wide area network (WAN), the internet, and such. The network 206 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 208 may include a variety of network devices, including routers, bridges, servers, computing devices, storage devices. The network devices within the network 208 may interact with the system 202 through communication links.

As discussed above, the system 202 may be implemented in a computing device 204, such as a hand-held device, a laptop or other portable computer, a tablet computer, a mobile phone, a PDA, a smartphone, and a desktop computer. The system 202 may also be implemented in a workstation, a mainframe computer, a server, and a network server. In an embodiment, the system 202 may be coupled to a data repository, for example, a repository 212. The repository 212 may store data processed, received, and generated by the system 202. In an alternate embodiment, the system 202 may include the data repository 212.

The network implementation 200 supports various connectivity options such as BLUETOOTH®, USB, ZigBee and other cellular services. The network environment enables connection of devices 206 such as Smartphone with the server 204, and accordingly with the database 212 using any communication link including Internet, WAN, MAN, and so on. In an exemplary embodiment, the system 202 is implemented to operate as a stand-alone device. In another embodiment, the system 202 may be implemented to work as a loosely coupled device to a smart computing environment.

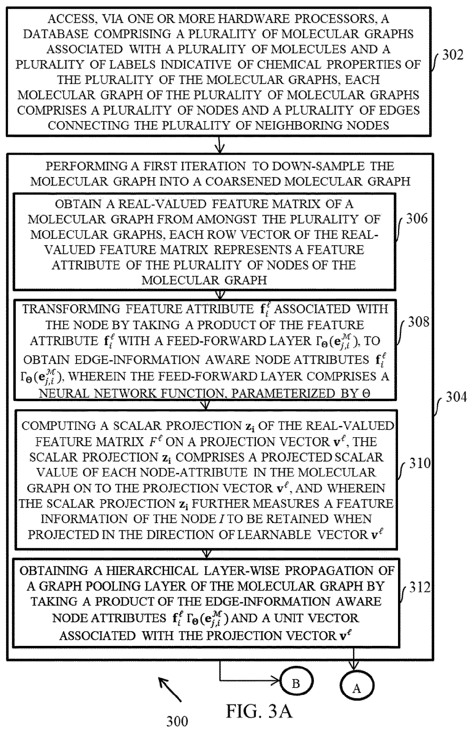

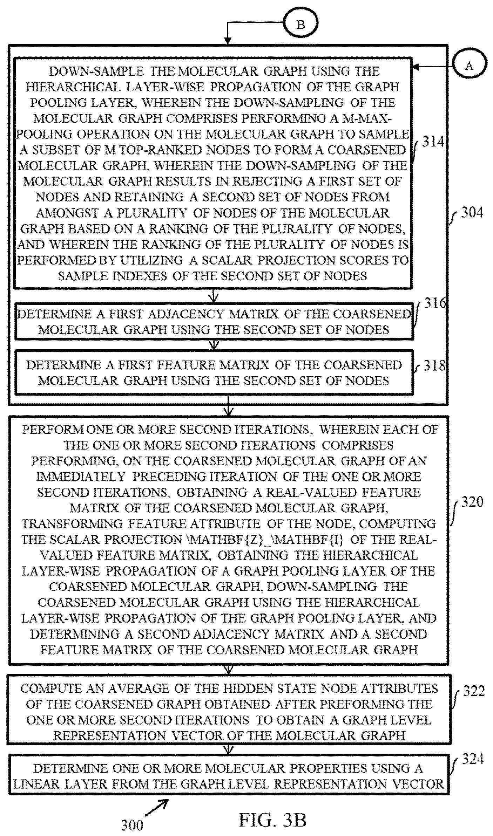

FIGS. 3A and 3B illustrate a flow chart of a method 300 for molecular property prediction using edge conditioned identity mapping convolution neural network, in accordance with an example embodiment of present disclosure. Operations of the flowchart, and combinations of operation in the flowchart, may be implemented by various means, such as hardware, firmware, processor, circuitry and/or other device associated with execution of software including one or more computer program instructions. For example, one or more of the procedures described in various embodiments may be embodied by computer program instructions. In an example embodiment, the computer program instructions, which embody the procedures, described in various embodiments may be stored by at least one memory device of a system and executed by at least one processor in the system. Any such computer program instructions may be loaded onto a computer or other programmable system (for example, hardware) to produce a machine, such that the resulting computer or other programmable system embody means for implementing the operations specified in the flowchart. It will be noted herein that the operations of the method 300 are described with help of system 202. However, the operations of the method 300 can be described and/or practiced by using any other system.

At step 302 of method 300, a database comprising a plurality of molecular graphs associated with a plurality of molecules is accessed, via one or more hardware processors. The database further includes a plurality of labels indicative of chemical properties of the plurality of the molecular graphs. Each molecular graph of the plurality of molecular graphs includes a plurality of nodes and a plurality of edges connecting a plurality of neighboring nodes from amongst the plurality of nodes. For example, the database may include annotated independent and identically distributed molecular graphs,

$$D^{\mathcal{M}} = (G_1^{\mathcal{M}}, y_1^{\mathcal{M}}), (G_1^{\mathcal{M}}, y_1^{\mathcal{M}}) \dots (G_{|D\mathcal{M}|}^{\mathcal{M}}, y_{|D\mathcal{M}|}^{\mathcal{M}})$$

Here,

$$y_i^{\mathcal{M}}$$

are the associated chemical properties corresponding to the molecular graph,

$$\mathcal{G}_A^{\mathcal{M}}.$$

The objective of the graph-based molecular property prediction framework is by operating on the topology of the molecular graphs described by a set of node features,

$$N^{\mathcal{M}} \in \mathbb{R}^{|\mathcal{V}^{\mathcal{M}}| \times c}$$

and static edge features,

$$e_{ij}^{\mathcal{M}} \in \mathbb{R}^{|\mathcal{E}^{\mathcal{M}}| \times z}, \forall (i,j) \in \mathcal{E}^{\mathcal{M}}$$

is to learn a novel mapping

$$f : \mathcal{G}^{\mathcal{M}} \to h_k^{\mathcal{M}}, \forall k \in \mathcal{V}^{\mathcal{M}} \to h_{\mathcal{G}}^{\mathcal{M}} \to y_i^{\mathcal{M}}$$

that maps molecular graphs structure to the set of labels.

$$h_k^{\mathcal{M}}$$

is the learned hidden representation vector of node k. Here,

$$y_i^{\mathcal{M}}$$

denotes the target molecular properties.

Herein, the molecular graph is iteratively down-sampled in a plurality of iterations to obtain a coarsened graph in each iteration. For example, in a first iteration of the plurality of iterations, down-sample the molecular graph is down sampled into a coarsened molecular graph at step 304. Subsequently, in one or more second iterations of the plurality of iterations, the coarsened molecular graph is further down sampled to further obtain a further coarsened molecular graph (as will be explained further with reference to step 318 of the method 300). Hence, in each iteration, the coarsened graph of previous iteration is taken an input and is further down sampled. The method 304 of down sampling the molecular graph to form the coarsened molecular graph is described further with reference to steps 306-318. Herein the number of iterations may be predetermined.

At 306, the method 300 includes obtaining, via the one or more hardware processors, a real-valued feature matrix of a molecular graph from amongst the plurality of molecular graphs, where each row vector of the real-valued feature matrix represents a feature attribute of the plurality of nodes of the molecular graph.

For the input graph, the real-valued feature matrix is represented by

$$F^{\ell} \equiv N^{\mathcal{M}} \in \mathbb{R}^{|\mathcal{V}^{\mathcal{M}}| \times c}$$

The superscript $\ell$ refers to a $l_{th}$ layer. Each row vector

$$\mathbf{f}_i^{\ell} \mathbb{R}^{1 \times c}$$

in $F^{\ell}$ denotes the node $i \in \mathcal{V}^{\mathcal{M}}$ feature attribute.

$$\left( \mathbf{f}_1^{\ell}, \mathbf{f}_2^{\ell}, \cdots, \mathbf{f}_{|\mathcal{V}^{\mathcal{M}}|}^{\ell} \right)$$

represents the node-level attributes in the molecular graph (or chemical graph). For performing the down-sampling of the chemical graph and to extract graph global-information, the hierarchical layer-wise propagation of the graph pooling layer is described by,

$$\mathbf{z} = \frac{F^{\ell} \mathbf{v}_i^{\ell}}{\| \mathbf{v}^{\ell} \|}$$

At 308, the method 300 includes transforming, via the one or more hardware processors, feature attribute $\mathbf{f}_i^{\ell}$ associated with the node by taking a product of the feature attribute $\mathbf{f}_i^{\ell}$ with a feed-forward layer

$$\Gamma_{\Theta}(\mathbf{e}_{j,i}^{\mathcal{M}}),$$

to obtain edge-information aware node attributes

$$\mathbf{f}_i^{\ell} \, \Gamma_{\Theta}(\mathbf{e}_{j,i}^{\mathcal{M}}).$$

Herein the feed-forward layer includes a neural network function, parameterized by $\Theta$.

At 310, the method 300 includes computing, via the one or more hardware processors, a scalar projection $\mathbf{z}_i$ of the real-valued feature matrix $F^{\ell}$ on a projection vector $\mathbf{v}^{\ell}$ resulting in $[z_1, z_2, \cdots, z_{|\mathcal{V}^{\mathcal{M}}|}]$. The scalar projection $\mathbf{z}_i$ measures a projected scalar value of each node-attribute in the molecular graph on to the projection vector $\mathbf{v}^{\ell}$. Also, the scalar projection $\mathbf{z}_i$ measures a feature information of the node i to be retained when projected in the direction of learnable vector $\mathbf{v}^{\ell}$.

Here, a hierarchical layer-wise propagation of a graph pooling layer permits to consider both the node attributes and graph topology to perform down-sampling on the molecular graphs. It can be leveraged on low-tree width graphs, regardless of the varying input molecular graph, $\mathcal{V}^{\mathcal{M}}$ ($|\mathcal{V}^{\mathcal{M}}|$) and $\mathcal{E}^{\mathcal{M}}$ ($|\mathcal{E}^{\mathcal{M}}|$). At 312, the method 300 includes obtaining the hierarchical layer-wise propagation of a graph pooling layer of the molecular graph by taking a product of the edge-information aware node attributes

$$\mathbf{f}_i^{\ell}\,\Gamma_{\Theta}\left(\mathbf{e}_{j,i}^{\mathcal{M}}\right)$$

and a unit vector associated with the projection vector $\mathbf{v}^{\ell}$. The unit vector in the same direction as $\mathbf{v}^{\ell}$, is given by $\mathbf{v}^{\ell}/\|\mathbf{v}^{\ell}\|$. The hierarchical layer-wise propagation of the graph pooling layer is given by:

$$\mathbf{z_i}^{\ell} = \frac{\mathbf{f}_i^{\ell}\Gamma_{\Theta}\left(\mathbf{e}_{j,i}^{\mathcal{M}}\right)\cdot\mathbf{v}_i^{\ell}}{\sqrt{\mathbf{v}_j^{\ell}\mathbf{v}_j^{\ell}}}$$

At 314, the method 300 includes down-sampling the molecular graph using the hierarchical layer-wise propagation of the graph pooling layer, via the one or more hardware processors. The down-sampling of the molecular graph includes performing a m-max-pooling operation on the molecular graph to sample a subset of m top-ranked nodes to form a coarsened molecular graph. Herein, the down-sampling of the molecular graph results in rejecting a first set of nodes (

$$|\mathcal{V}^{\mathcal{M}}| - \lceil p_r|\mathcal{V}^{\mathcal{M}}|\rceil$$

) and retaining a second set of nodes (

$$\lceil p_r|\mathcal{V}^{\mathcal{M}}|\rceil$$

) from amongst a plurality of nodes of the molecular graph based on a ranking of the plurality of nodes. Further herein, the ranking of the plurality of nodes is performed by utilizing a scalar projection scores to sample indexes of the second set of nodes. Selection of the first and the second set of nodes is done based on a pooling ratio defined by:

$$p_r = \frac{m}{|\mathcal{V}^{\mathcal{M}}|}, \forall\, p_r \in (0,1]$$

Herein, the node ranking operation, $ind = rank(z^{\ell}, m)$ utilizes the scalar projection scores, $z^{\ell}$ to sample a subset of m-prominent node indexes (or the indexes of the second set of nodes). It augments the node-local receptive field and enables the high-level feature information encoding by performing graph-pooling on varying input graph sizes.

At 316, the method 300 includes determining, via the one or more hardware processors, a first adjacency matrix of the coarsened molecular graph using the second set of nodes. For example, let the m-selected indices of the nodes, i.e. the second set of nodes are $j_1, j_2, \cdots, j_m$ with $j_p < j_q$ and $1 < p < q \leq m$. The selection of the indexes retains the seleced nodes

$$\lceil p_r|\mathcal{V}^{\mathcal{M}}|\rceil$$

feature information from the original molecular graph. It is permutation equivariant,

$$\mathcal{F}\left(P\mathcal{G}_A^{\mathcal{M}}P^{\top}\right) = P\mathcal{F}\left(\mathcal{G}_A^{\mathcal{M}}\right)$$

and P is a permutation matrix.

$$\mathcal{G}_A^{\ell}(\,idx\,,idx\,)$$

performs the row and column extraction to determine the adjacency matrix of the resulted coarsened chemical graph

$$\mathcal{G}_A^{\ell+1} \in \mathbb{R}^{m \times m}.$$

At 318, the method 300 includes determining, via the one or more hardware processors, a first feature matrix of the coarsened molecular graph using the second set of nodes. $\tilde{F}^{\ell} = F^{\ell}(\,idx\,,:)$ denotes the feature matrix of the coarsened molecular graph

$$\tilde{F}^{\ell} \in \mathbb{R}^{m \times c}.$$

. Herein, each row of the first feature matrix corresponds to hidden state node attributes of the coarsened molecular graph.

At 320, the method 300 includes performing one or more second iterations, via the one or more hardware processors. Each of the one or more second iterations include performing a number of steps to down-sample a coarsened molecular graph of an immediately preceding iteration of the one or more second iterations. For example, in the subsequent iterations, for instance one or more second iterations, the steps of obtaining a real-valued feature matrix of the coarsened molecular graph, transforming feature attribute of the node, computing the scalar projection $z_i$ of the real-valued feature matrix, obtaining the hierarchical layer-wise propagation of a graph pooling layer of the coarsened molecular graph, down-sampling the coarsened molecular graph using the hierarchical layer-wise propagation of the graph pooling layer, and determining a second adjacency matrix and a second feature matrix of the coarsened molecular graph, are repeated. A count of the one or more second iterations may be predetermined.

At 322, the method 300 includes computing, via the one or more hardware processors, an average of the hidden state node attributes of the coarsened graph obtained after preforming the one or more second iterations to obtain a graph level representation vector of the molecular graph. In an embodiment, obtaining the graph level representation vector of the molecular graph includes performing a spatial-graph convolution on the coarsened molecular graph to transform and update each of the hidden state node attributes. An additional predetermined number of down-sampling and subsequent spatial-graph convolution on the molecular graph is performed and an equivalent number of up-sampling and subsequent spatial-graph convolution are performed to reinstate the coarsened molecular graph to an isomorphic clone of the molecular graph. The isomorphic clone of the molecular graph is a node-information transformed isomorphic clone. Thereafter, the node-information transformed isomorphic clone of the molecular graph is fed as an input to a node-ordering invariant read-out function to determine the graph-level representation vector. In the present embodiment, The spatial graph convolution takes as input each molecular graph from a plurality of molecular graphs. Each node in the molecular graph during spatial convolution receives and dispatches feature information embedded local-graph messages to its respective local-graph neighbors. Each node in the molecular graph transforms its hidden state based on the neural-information messages received perceived from its local-neighborhood.

In another embodiment, the graph level representation vector of the molecular graph includes is obtained by performing spatial dynamic neighborhood aggregation on the coarsened molecular graph to transform and update each of the hidden state node attributes. Further, an additional predetermined number of down-sampling and subsequent spatial dynamic neighborhood aggregation on the molecular graph is performed, and an equivalent number of up-sampling and subsequent spatial dynamic neighborhood aggregation is performed to reinstate the coarsened molecular graph to an isomorphic clone of the molecular graph. The isomorphic clone of the molecular graph is a node-information transformed isomorphic clone. Thereafter, the node-information transformed isomorphic clone of the molecular graph is fed as an input to a node-ordering invariant read-out function to determine the graph-level representation vector. In the present embodiment, the spatial-dynamic neighborhood aggregation transforms each node hidden state attribute by revisiting the previous iteration step local-graph neighborhood hidden states during the message-passing phase through attention mechanism.

In yet another embodiment, obtaining the graph level representation vector of the molecular graph includes performing spatial graph-attention feed-forward propagation layer mechanism on the coarsened molecular graph to transform and update each of the hidden state node attributes. An additional predetermined number of down-sampling and subsequent spatial graph-attention feed-forward propagation layer mechanism is performed on the molecular graph. Further, an equivalent number of up-sampling and subsequent spatial graph-attention feed-forward propagation layer mechanism are performed to reinstate the coarsened molecular graph to an isomorphic clone of the molecular graph. The isomorphic clone of the molecular graph is a node-information transformed isomorphic clone. Finally, the node-information transformed isomorphic clone of the molecular graph is fed as an input to a node-ordering invariant read-out function to determine the graph-level representation vector. Herein, the spatial- graph-attention feed-forward propagation layer mechanism overhauls each node hidden state attribute by weighing local-graph neighborhood nodes of importance. The local-graph neighborhood nodes' importance to the sink node is determined through the attention mechanism.

In still another embodiment, the graph level representation vector of the molecular graph includes performing spatial-Identity Mapping Convolution Networks on the coarsened molecular graph to transform and update each of the hidden state node attributes. Further, an additional predetermined number of down-sampling and subsequent spatial-Identity Mapping Convolution Networks on the molecular graph is performed. An equivalent number of up-sampling and subsequent spatial graph-attention feed-forward propagation layer mechanism are performed to reinstate the coarsened molecular graph to an isomorphic clone of the molecular graph. The isomorphic clone of the molecular graph is a node-information transformed isomorphic clone. The node-information transformed isomorphic clone of the molecular graph is fed as an input to a node-ordering invariant read-out function to determine the graph-level representation vector. **In** the present embodiment, the spatial-Identity Mapping Convolution Networks overhauls each node attribute by perceiving the neural messages from its local graph neighbors. The neural messages characterize the local-graph neighborhood feature information transformed by the edge attributes as given by the local-graph connectivity.

In still another embodiment, obtaining the graph level representation vector of the molecular graph includes performing spatial Graph Attentional Propagation on the coarsened molecular graph to transform and update each of the hidden state node attributes. An additional predetermined number of down-sampling and subsequent spatial Graph Attentional Propagation on the molecular graph is performed. Further, an equivalent number of up-sampling and subsequent spatial Graph Attentional Propagation is performed to reinstate the coarsened molecular graph to an isomorphic clone of the molecular graph. The isomorphic clone of the molecular graph is a node-information transformed isomorphic clone. Finally, the node-information transformed isomorphic clone of the molecular graph is fed as an input to a node-ordering invariant read-out function to determine the graph-level representation vector. In the present embodiment, the Spatial-Graph Attentional Propagation mechanism transforms the hidden state attribute of each node by determining the local-graph neighborhood nodes of importance through attention mechanism. The cosine similarity is the attention mechanism to determine the attention coefficients to weigh nodes of importance to transform each node attribute.

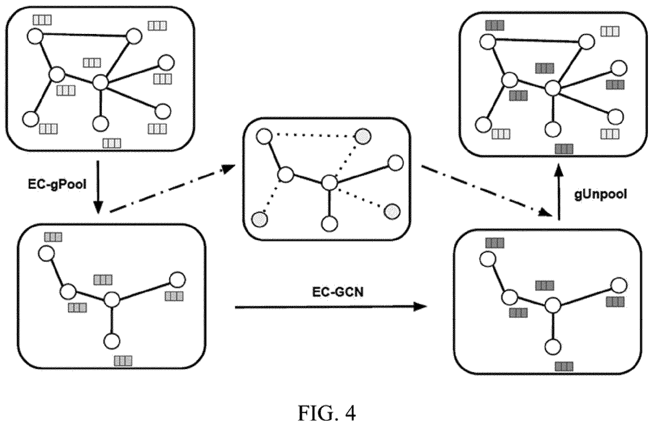

At 324, the method 300 includes determining one or more molecular properties using a linear layer from the graph level representation vector, via the one or more hardware processors. An example of a molecular graph being down sampled for molecular property prediction is illustrated with reference to FIG. 4.

U-Nets is an encoder-decoder architecture based on ConvNets for biomedical image segmentation tasks. The encoder of the encoder-decoder neural-network framework is utilized to reduce the feature map size and augment the receptive field. Whereas in the case of decoders, feature maps need to be up-sampled to reinstate the initial resolutions. The gUnpool layer accomplishes the unpooling operations on graph-structured data. Graph Unpooling layer implements the converse operation to be in agreement with gPool layer by leveraging the indexes of the retained and dropped vertices in the native graph, to reinstate the coarsened graph into it an isomorphic clone of the input graph structure. The layer-wise forward propagation of graph unpooling mechanism is described by,

$$F^{\ell+1} = distribute \ (0_{|\mathcal{V}^{\mathcal{M}}|xc}, F^{\ell}, ind)$$

The selected indexes of the nodes obtained after applying the down-sampling (gPool) mechanism that shrinks the input graph size from $|\mathcal{V}^{\mathcal{M}}|$ nodes to $m$ nodes is given by $ind \in Z^{m}$.

$$F^{\ell} \in \mathbb{R}^{mxc}$$

represents the node attributes of the coarsened graph. $0_{|\mathcal{V}^{\mathcal{M}}|xc}$ is a feature information matrix for the graph formed after applying gUnpool layer to a coarsened graph. Distribute ($0_{|\mathcal{V}^{\mathcal{M}}|xc}$, $F^{\ell}$ $ind$) (is the mathematical operation that replicates the each node attribute corresponding row vectors in $F^{\ell}$ towards into the $0_{|\mathcal{V}^{\mathcal{M}}|xc}$ feature information matrix acceding to their corresponding indexes indicated by ind. In the feature information matrix $F^{\ell+1}$, row vectors with indices given by, ind are overhauled by the corresponding row vectors in $F^{\ell}$. The rest of the row vectors are zero.

As illustrated in FIG. 4, the molecular graph 400 is characterized by $N(|\mathcal{V}|) = 8$ and $\varepsilon(\varepsilon = 8)$, it is down-sampled by leveraging a gPool layer in an encoder block. It results in a coarsened graph comprising of fewer retained vertices from the original input graph. The indexes of the selected vertices are noted. EC-GCN performs neural-message pooling from the each-node local-graph neighborhood and transforms its node embeddings. Thereafter, a corresponding gUnpool layer utilizes the selected and unselected node indexes aware-knowledge to retrieve the initial graph.

An algorithm for the proposed method is shown below:

> **Require**: Molecular Graph GM
>
> Apply dropout on *data.edgeindex, data.edge - attr*
>
> Apply dropout on *data.x*
>
> \# Message-Passing Phase
>
> **for** $t \leq T$ do
>
> Message-Propagation, EC-IMCN
>
> Vertex Update, GRU
>
> **end for**
>
> \# Addon Message-Passing Phase
>
> Edge-Conditioned Graph U-Net
>
> \# Read out Operation
>
> Graph Structure Incorporated Set2Set Algorithm
>
> Linear Layer
>
> **Return** Predicted Molecular Properties

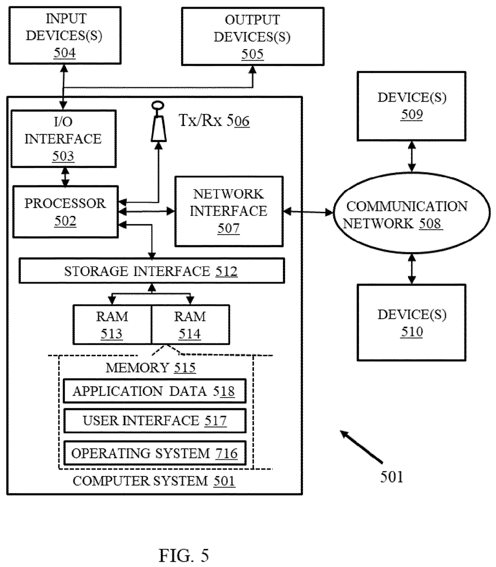

FIG. 5 is a block diagram of an exemplary computer system 501 for implementing embodiments consistent with the present disclosure. The computer system 501 may be implemented in alone or in combination of components of the system 202 (FIG. 2). Variations of the computer system 501 may be used for implementing the devices included in this disclosure. The computer system 501 may comprise a central processing unit ("CPU" or "hardware processor" or "processor") 502. The hardware processor 502 may comprise at least one data processor for executing program components for executing user- or system-generated requests. The processor may include specialized processing units such as integrated system (bus) controllers, memory management control units, floating point units, graphics processing units, digital signal processing units, etc. The processor may include a microprocessor, such as AMD Athlon™, Duron™ or Opteron™, ARM's application, embedded or secure processors, IBM PowerPC™, Intel's Core, Itanium™, Xeon™, Celeron™ or other line of processors, etc. The processor 502 may be implemented using mainframe, distributed processor, multi-core, parallel, grid, or other architectures. Some embodiments may utilize embedded technologies like application specific integrated circuits (ASICs), digital signal processors (DSPs), Field Programmable Gate Arrays (FPGAs), etc. The processor 502 may be a multi-core multi-threaded processor.

The processor 502 may be disposed in communication with one or more input/output (I/O) devices via I/O interface 503. The I/O interface 503 may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.11 a/b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface 503, the computer system 501 may communicate with one or more I/O devices. For example, the input device 504 may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, gyroscope, proximity sensor, or the like), stylus, scanner, storage device, transceiver, video device/source, visors, etc.

Output device 505 may be a printer, fax machine, video display (e.g., cathode ray tube (CRT), liquid crystal display (LCD), light-emitting diode (LED), plasma, or the like), audio speaker, etc. In some embodiments, a transceiver 506 may be disposed in connection with the processor 502. The transceiver may facilitate various types of wireless transmission or reception. For example, the transceiver may include an antenna operatively connected to a transceiver chip (e.g., Texas Instruments WiLink WL1283, Broadcom BCM4750IUB8, Infineon Technologies X-Gold 618-PMB9800, or the like), providing IEEE 802.11a/b/g/n, Bluetooth, FM, global positioning system (GPS), 2G/3G HSDPA/HSUPA communications, etc.

In some embodiments, the processor 502 may be disposed in communication with a communication network 508 via a network interface 507. The network interface 507 may communicate with the communication network 508. The network interface may employ connection protocols including, without limitation, direct connect, Ethernet (e.g., twisted pair 10/100/1000 Base T), transmission control protocol/internet protocol (TCP/IP), token ring, IEEE 802.11a/b/g/n/x, etc. The communication network 508 may include, without limitation, a direct interconnection, local area network (LAN), wide area network (WAN), wireless network (e.g., using Wireless Application Protocol), the Internet, etc. Using the network interface 507 and the communication network 508, the computer system 501 may communicate with devices 509 and 510. These devices may include, without limitation, personal computer(s), server(s), fax machines, printers, scanners, various mobile devices such as cellular telephones, smartphones (e.g., Apple iPhone, Blackberry, Android-based phones, etc.), tablet computers, eBook readers (Amazon Kindle, Nook, etc.), laptop computers, notebooks, gaming consoles (Microsoft Xbox, Nintendo DS, Sony PlayStation, etc.), or the like. In some embodiments, the computer system 501 may itself embody one or more of these devices.

In some embodiments, the processor 502 may be disposed in communication with one or more memory devices (e.g., RAM 513, ROM 514, etc.) via a storage interface 512. The storage interface may connect to memory devices including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magneto-optical drive, optical drive, redundant array of independent discs (RAID), solid-state memory devices, solid-state drives, etc. Variations of memory devices may be used for implementing, for example, any databases utilized in this disclosure.

The memory devices may store a collection of programs or database components, including, without limitation, an operating system 516, a user interface application 517, a user/application data 518 (e.g., any data variables or data records discussed in this disclosure), etc. The operating system 516 may facilitate resource management and operation of the computer system 501. Examples of operating systems include, without limitation, Apple Macintosh OS X, Unix, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like. The user interface 517 may facilitate display, execution, interaction, manipulation, or operation of program components through textual or graphical facilities. For example, user interfaces may provide computer interaction interface elements on a display system operatively connected to the computer system 501, such as cursors, icons, check boxes, menus, scrollers, windows, widgets, etc. Graphical user interfaces (GUIs) may be employed, including, without limitation, Apple Macintosh operating systems' Aqua, IBM OS/2, Microsoft Windows (e.g., Aero, Metro, etc.), Unix X-Windows, web interface libraries (e.g., ActiveX, Java, Javascript, AJAX, HTML, Adobe Flash, etc.), or the like.

In some embodiments, the computer system 501 may store the user/application data 518, such as the data, variables, records, etc. as described in this disclosure. Such databases may be implemented as fault-tolerant, relational, scalable, secure databases such as Oracle or Sybase. Alternatively, such databases may be implemented using standardized data structures, such as an array, hash, linked list, structured text file (e.g., XML), table, or as hand-oriented databases (e.g., using HandStore, Poet, Zope, etc.). Such databases may be consolidated or distributed, sometimes among various computer systems discussed above. It is to be understood that the structure and operation of any computer or database component may be combined, consolidated, or distributed in any working combination.

Additionally, in some embodiments, (the server, messaging and instructions transmitted or received may emanate from hardware, including operating system, and program code (i.e., application code) residing in a cloud implementation. Further, it should be noted that one or more of the systems and methods provided herein may be suitable for cloud-based implementation. For example, in some embodiments, some or all of the data used in the disclosed methods may be sourced from or stored on any cloud computing platform.

Example:

For the purpose of validation, experiments were conducted using QM-9 data set.

The model (as implemented by the disclose system) was trained for graph-level-based regression tasks for the Quantum Chemistry property prediction task. Message passing computational steps, T was constrained to be at 3. The set2set mathematical iterations, M was on par with T. Here, the model trained by leveraging a random selection of datasets for stochastic gradient descent optimization with the Adaptive Moment Estimation optimizer algorithm, with batch size 10. The number of iterations(epochs) is 100 cycles through the full training dataset. The beginning learning rate was chosen as

$1e^{-3}$. The learning rate was decayed at 51st epoch by half and maintained it constant in the span of [51; 75] epochs throughout the training and the beginning step size learning rate I decayed to a terminating learning rate $2.5e^{-4}$, using a decay factor by 4 in the range [76; 100] epochs. The QM-9 dataset consists of approximately 134K molecules. The validation set comprises 10000 samples. The test set is composed of 10000 samples and the remaining are for the training set. Here, early stopping is implemented on the validation dataset to prevent the model from over-fitting and for model selection. Finally, the performance of the model was evaluated and the evaluation metric based totally on the test set was published. Feature scaling was performed on the target properties to be predicted. Z-score normalization is leveraged to have distribution mean zero and the expectation of the squared deviation to one for each target property. The gradient descent (aka back-propagation) algorithm was run in weight space by updating the parameters according to the gradients of the loss function, the mean squared error between the predicted model outputs and the predetermined DFT properties. The results are reported in MAE metric in the table below:

Table: Performance comparison of the disclosed method with the baseline algorithms on test dataset

| Target | Unit | PPGN | SchNet | PhysNet | MEGNets | Comorant | DimeNet | EC-GPL |
|--------|------|------|--------|---------|---------|----------|---------|--------|
| Cv | cal/(mol K) | 0.055 | 0.033 | 0.0529 | 0.05 | 0.13 | 0.0286 | 0.0029 |
| G | meV | 36.4 | 14 | 9.4 | 12 | - | 8.98 | 0.0034 |
| H | meV | 36.3 | 14 | 8.42 | 12 | - | 8.11 | 0.0108 |
| HOMO | meV | 40.3 | 41 | 32.9 | 43 | 36 | 27.8 | 0.0102 |
| LUMO | meV | 32.7 | 34 | 24.7 | 44 | 36 | 19.7 | 0.0143 |
| R2 | Bohr2 | 0.592 | 0.073 | 0.765 | 0.302 | 0.673 | 0.331 | 0.0291 |
| U | meV | 36.8 | 14 | 8.15 | 12 | - | 7.89 | 0.0227 |
| U0 | meV | 36.8 | 14 | 8.15 | 12 | - | 8.02 | 0.0910 |
| ZPVE | meV | 3.12 | 1.7 | 1.39 | 1.43 | 1.98 | 1.29 | 0.0001417 |
| alpha | Bohr3 | 0.131 | 0.235 | 0.0615 | 0.081 | 0.092 | 0.0469 | 0.0368 |
| gap | meV | 60 | 63 | 42.5 | 66 | 60 | 34.8 | 0.0521 |
| mu | D | 0.047 | 0.033 | 0.0529 | 0.05 | 0.13 | 0.0286 | 0.0114 |

The written description describes the subject matter herein to enable any person skilled in the art to make and use the embodiments. The scope of the subject matter embodiments is defined by the claims.

Various embodiments described herein method and system for molecular property predictions. The disclosed system utilizes an Edge-Conditioned Hierarchical Graph Pooling Layer (EC-GPL). In particular, the encoder of an Edge-Conditioned Graph U-Nets Architecture, consists of quite a few stack of encoding blocks. Each encoding block is composed of a downsampling(gPool) layer and an Edge-Conditioned Graph Convolutional Network (EC-GCN) layer. gPool layers reduce the feature map size by encoding higher-order features and augments the receptive field. EC-GCN transforms the node embeddings of the coarsened graph by neural-message passing schemes which encapsulate the hierarchical structure by exploiting the topology of the graph. To augment the model performance, the neural-embedded messages are aggregated from neighboring nodes at a route of length 2(2 hops) in the graph. Each encoding block transforms initial node attributes, $\in \mathbb{R}^{|V^{\mathcal{M}}| \times C} \left( \mathbf{h}_i^0 = N_i^{\mathcal{M}}, i \in V^{\mathcal{M}} \right)$ to vector embeddings, $\in \mathbb{R}^{\mathcal{M} \times C}$. Here, G - ENC: $\mathbb{R}^{|V^{\mathcal{M}}| \times C} \to \mathbb{R}^{m \times C}$. Similarly, the decoder part of the Edge- Conditioned Graph U-Nets Architecture consists of quite a few stacks of a similar number of decoding blocks in comparison with the encoding blocks comprising the encoder. Every decoder block consists of an Upsampling(gUnpool) layer and then accompanied subsequently by a forward propagation, EC-GCN layer. Here, the Upsampling mechanism reinstates the coarsened graph to the isomorphic clone of the input graph. Each decoding block transforms node embeddings, G - Dec, $\mathbb{R}^{m \times C} \to \mathbb{R}^{|V^{\mathcal{M}}| \times C}$. There exist skipconnections in the proposed architecture to pass on the node-attributes information by feature map summation between the resembling and coinciding blocks of encoder and decoder layers on different granularities of the input coarsened graph. These skip connections retain the neural embedded information of the nodes in the graph obtained from the former intermediate message passing schemes.

The scope of the disclosure extends to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300), comprising:

   accessing (302), via one or more hardware processors, a database comprising a plurality of molecular graphs associated with a plurality of molecules and a plurality of labels indicative of chemical properties of the plurality of the molecular graphs, wherein each molecular graph of the plurality of molecular graphs comprises a plurality of nodes and a plurality of edges connecting a plurality of neighboring nodes;
   performing (304), via the one or more hardware processors, a first iteration to down-sample a molecular graph from amongst the plurality of molecular graph into a coarsened molecular graph, the first iteration comprising:

   obtaining (306) a real-valued feature matrix of a molecular graph from amongst the plurality of molecular graphs, each row vector of the real-valued feature matrix represents a feature attribute of the plurality of nodes of the molecular graph;

   transforming (308) feature attribute $\mathbf{f}_i^{\ell}$ associated with the node by taking a product of the feature attribute $\mathbf{f}_i^{\ell}$ with a feed-forward layer $\Gamma_\Theta(\mathbf{e}_{j,i}^{\mathcal{M}})$, to obtain edge-information aware node attributes $\mathbf{f}_i^{\ell}\,\Gamma_\Theta(\mathbf{e}_{j,i}^{\mathcal{M}})$, wherein the feed-forward layer comprises a neural network function, parameterized by $\Theta$;

computing (310) a scalar projection $z_i$ of the real-valued feature matrix $F^\ell$ on a projection vector $\mathbf{v}^\ell$, the scalar projection $z_i$ comprises a projected scalar value of each node-attribute in the molecular graph on to the projection vector $\mathbf{v}^\ell$, and wherein the scalar projection $z_i$ further measures a feature information of the node i to be retained when projected in the direction of learnable vector $\mathbf{v}^\ell$;

obtaining (312) a hierarchical layer-wise propagation of a graph pooling layer of the molecular graph by taking a product of the edge-information aware node attributes $\mathbf{f}_i^\ell \ \Gamma_\Theta\left(\mathbf{e}_{j,i}^{\mathcal{H}\ell}\right)$ and a unit vector associated with the projection vector $\mathbf{v}^\ell$ wherein the direction of the unit vector is same as the direction of the projection vector $\mathbf{v}^\ell$;

down-sampling (314) the molecular graph using the hierarchical layer-wise propagation of the graph pooling layer, wherein the down-sampling of the molecular graph comprises performing a m-max-pooling operation on the molecular graph to sample a subset of m top-ranked nodes to form a coarsened molecular graph, wherein the down-sampling of the molecular graph results in rejecting a first set of nodes and retaining a second set of nodes from amongst a plurality of nodes of the molecular graph based on a ranking of the plurality of nodes, and wherein the ranking of the plurality of nodes is performed by utilizing a scalar projection scores to sample indexes of the second set of nodes;

determining (316) a first adjacency matrix of the coarsened molecular graph using the second set of nodes; and

determining (318) a first feature matrix of the coarsened molecular graph using the second set of nodes, wherein each row of the first feature matrix corresponds to hidden state node attributes of the coarsened molecular graph;

performing (320), via the one or more hardware processors, one or more second iterations, wherein each of the one or more second iterations comprises performing, on the coarsened molecular graph of an immediately preceding iteration of the one or more second iterations, obtaining a real-valued feature matrix of the coarsened molecular graph, transforming feature attribute of the node, computing the scalar projection $z_i$ of the real-valued feature matrix, obtaining the hierarchical layer-wise propagation of a graph pooling layer of the coarsened molecular graph, down-sampling the coarsened molecular graph using the hierarchical layer-wise propagation of the graph pooling layer, and determining a second adjacency matrix and a second feature matrix of the coarsened molecular graph; and

computing (322), via the one or more hardware processors, an average of the hidden state node attributes of the coarsened molecular graph obtained after preforming the one or more second iterations to obtain a graph level representation vector of the molecular graph, wherein a linear layer from the graph level representation vector assists in inductive learning tasks for molecular property prediction by reducing computational complexity.

2. The processor implemented method of claim 1, wherein obtaining the graph level representation vector of the molecular graph comprises:

performing spatial-graph convolution on the coarsened molecular graph to transform and update each of the hidden state node attributes;

performing an additional predetermined number of down-sampling and subsequent spatial-graph convolution on the molecular graph;

performing equivalent number of up-sampling and subsequent spatial-graph convolution to reinstate the coarsened molecular graph to an isomorphic clone of the molecular graph, wherein the isomorphic clone of the molecular graph is a node-information transformed isomorphic clone; and

feeding the node-information transformed isomorphic clone of the molecular graph as an input to a node-ordering invariant read-out function to determine the graph-level representation vector.

3. The method of claim 1, wherein obtaining the graph level representation vector of the molecular graph comprises:

performing spatial dynamic neighborhood aggregation on the coarsened molecular graph to transform and update each of the hidden state node attributes;

performing an additional predetermined number of down-sampling and subsequent spatial dynamic neighborhood aggregation on the molecular graph;

performing equivalent number of up-sampling and subsequent spatial dynamic neighborhood aggregation to reinstate the coarsened molecular graph to an isomorphic clone of the molecular graph, wherein the isomorphic clone of the molecular graph is a node-information transformed isomorphic clone; and

feeding the node-information transformed isomorphic clone of the molecular graph as an input to a node-ordering invariant read-out function to determine the graph-level representation vector.

4. The method of claim 1, wherein obtaining the graph level representation vector of the molecular graph comprises:

> performing spatial graph-attention feed-forward propagation layer mechanism on the coarsened molecular graph to transform and update each of the hidden state node attributes;
> performing an additional predetermined number of down-sampling and subsequent spatial graph-attention feed-forward propagation layer mechanism on the molecular graph;
> performing equivalent number of up-sampling and subsequent spatial graph-attention feed-forward propagation layer mechanism to reinstate the coarsened molecular graph to an isomorphic clone of the molecular graph, wherein the isomorphic clone of the molecular graph is a node-information transformed isomorphic clone; and
> feeding the node-information transformed isomorphic clone of the molecular graph as an input to a node-ordering invariant read-out function to determine the graph-level representation vector.

5. The method of claim 1, wherein obtaining the graph level representation vector of the molecular graph comprises:

> performing spatial-Identity Mapping Convolution Networks on the coarsened molecular graph to transform and update each of the hidden state node attributes;
> performing an additional predetermined number of down-sampling and subsequent spatial-Identity Mapping Convolution Networks on the molecular graph;
> performing equivalent number of up-sampling and subsequent spatial graph-attention feed-forward propagation layer mechanism to reinstate the coarsened molecular graph to an isomorphic clone of the molecular graph, wherein the isomorphic clone of the molecular graph is a node-information transformed isomorphic clone; and
> feeding the node-information transformed isomorphic clone of the molecular graph as an input to a node-ordering invariant read-out function to determine the graph-level representation vector.

6. The method of claim 1, wherein obtaining the graph level representation vector of the molecular graph comprises:

> performing spatial Graph Attentional Propagation on the coarsened molecular graph to transform and update each of the hidden state node attributes;
> performing an additional predetermined number of down-sampling and subsequent spatial Graph Attentional Propagation on the molecular graph;
> performing equivalent number of up-sampling and subsequent spatial Graph Attentional Propagation to reinstate the coarsened molecular graph to an isomorphic clone of the molecular graph, wherein the isomorphic clone of the molecular graph is a node-information transformed isomorphic clone; and
> feeding the node-information transformed isomorphic clone of the molecular graph as an input to a node-ordering invariant read-out function to determine the graph-level representation vector.

7. A system (500), comprising:

> a memory (515) storing instructions;
> one or more communication interfaces (503); and
> one or more hardware processors (502) coupled to the memory (515) via the one or more communication interfaces (503), wherein the one or more hardware processors (502) are configured by the instructions to:

>> access a database comprising a plurality of molecular graphs associated with a plurality of molecules and a plurality of labels indicative of chemical properties of the plurality of the molecular graphs, wherein each molecular graph of the plurality of molecular graphs comprises a plurality of nodes and a plurality of edges connecting a plurality of neighboring nodes;
>> perform a first iteration to down-sample the molecular graph into a coarsened molecular graph, wherein to perform the first iteration the one or more hardware processors are configured by the instructions to:

>>> obtain a real-valued feature matrix of a molecular graph from amongst the plurality of molecular graphs, each row vector of the real-valued feature matrix represents a feature attribute of the plurality of nodes of the molecular graph;

>>> transform feature attribute $\mathbf{f}_i^{\ell}$ associated with the node by taking a product of the feature attribute $\mathbf{f}_i^{\ell}$ with a feed-forward layer

$$\Gamma_{\Theta}(\mathbf{e}_{j,i}^{\mathcal{M}}),$$

to obtain edge-information aware node attributes

$$\mathbf{f}_i^{\ell} \, \Gamma_{\Theta}(\mathbf{e}_{j,i}^{\mathcal{M}}),$$

wherein the feed-forward layer comprises a neural network function, parameterized by $\Theta$;
compute a scalar projection $\mathbf{z}_i$ of the real-valued feature matrix $F^{\ell}$ on a projection vector $\mathbf{v}^{\ell}$, the scalar projection $\mathbf{z}_j$ comprises a projected scalar value of each node-attribute in the molecular graph on to the projection vector $\mathbf{v}^{\ell}$, and wherein the scalar projection $\mathbf{z}_i$ further measures a feature information of the node $i$ to be retained when projected in the direction of learnable vector $\mathbf{v}^{\ell}$;
obtain a hierarchical layer-wise propagation of a graph pooling layer of the molecular graph by taking a product of the edge-information aware node attributes

$$\mathbf{f}_i^{\ell} \, \Gamma_{\Theta}(\mathbf{e}_{j,i}^{\mathcal{M}})$$

and a unit vector associated with the projection vector $\mathbf{v}^{\ell}$, wherein the direction of the unit vector is same as the direction of the projection vector $\mathbf{v}^{\ell}$;
down-sample the molecular graph using the hierarchical layer-wise propagation of the graph pooling layer, wherein the down-sampling of the molecular graph comprises performing a m-max-pooling operation on the molecular graph to sample a subset of m top-ranked nodes to form a coarsened molecular graph, wherein the down-sampling of the molecular graph results in rejecting a first set of nodes and retaining a second set of nodes from amongst a plurality of nodes of the molecular graph based on a ranking of the plurality of nodes, and wherein the ranking of the plurality of nodes is performed by utilizing a scalar projection scores to sample indexes of the second set of nodes;
determine a first adjacency matrix of the coarsened molecular graph using the second set of nodes;
determine a first feature matrix of the coarsened molecular graph using the second set of nodes, wherein each row of the first feature matrix corresponds to hidden state node attributes of the coarsened molecular graph;

perform one or more second iterations, wherein each of the one or more second iterations comprises performing, on the coarsened molecular graph of an immediately preceding iteration of the one or more second iterations, obtaining a real-valued feature matrix of the coarsened molecular graph, transforming feature attribute of the node, computing the scalar projection $\mathbf{z}_i$ of the real-valued feature matrix, obtaining the hierarchical layer-wise propagation of a graph pooling layer of the coarsened molecular graph, down-sampling the coarsened molecular graph using the hierarchical layer-wise propagation of the graph pooling layer, and determining a second adjacency matrix and a second feature matrix of the coarsened molecular graph; and
compute an average of the hidden state node attributes of the coarsened molecular graph obtained after preforming the one or more second iterations to obtain a graph level representation vector of the molecular graph, wherein a linear layer from the graph level representation vector assists in inductive learning tasks for molecular property prediction by reducing computational complexity.

8. The system of claim 7, wherein to obtain the graph level representation vector of the molecular graph, the one or more hardware processors are configured by the instructions to:

perform spatial-graph convolution on the coarsened molecular graph to transform and update each of the hidden state node attributes;
perform an additional predetermined number of down-sampling and subsequent spatial-graph convolution on the molecular graph;
perform equivalent number of up-sampling and subsequent spatial-graph convolution to reinstate the coarsened molecular graph to an isomorphic clone of the molecular graph, wherein the isomorphic clone of the molecular graph is a node-information transformed isomorphic clone; and

feed the node-information transformed isomorphic clone of the molecular graph as an input to a node-ordering invariant read-out function to determine the graph-level representation vector.

9. The system of claim 7, wherein to obtain the graph level representation vector of the molecular graph, the one or more hardware processors are configured by the instructions to:

perform spatial dynamic neighborhood aggregation on the coarsened molecular graph to transform and update each of the hidden state node attributes;
perform an additional predetermined number of down-sampling and subsequent spatial dynamic neighborhood aggregation on the molecular graph;
perform equivalent number of up-sampling and subsequent spatial dynamic neighborhood aggregation to reinstate the coarsened molecular graph to an isomorphic clone of the molecular graph, wherein the isomorphic clone of the molecular graph is a node-information transformed isomorphic clone; and
feed the node-information transformed isomorphic clone of the molecular graph as an input to a node-ordering invariant read-out function to determine the graph-level representation vector.

10. The system of claim 7, wherein to obtain the graph level representation vector of the molecular graph, the one or more hardware processors are configured by the instructions to:

perform spatial graph-attention feed-forward propagation layer mechanism on the coarsened molecular graph to transform and update each of the hidden state node attributes;
perform an additional predetermined number of down-sampling and subsequent spatial graph-attention feed-forward propagation layer mechanism on the molecular graph;
perform equivalent number of up-sampling and subsequent spatial graph-attention feed-forward propagation layer mechanism to reinstate the coarsened molecular graph to an isomorphic clone of the molecular graph, wherein the isomorphic clone of the molecular graph is a node-information transformed isomorphic clone; and
feed the node-information transformed isomorphic clone of the molecular graph as an input to a node-ordering invariant read-out function to determine the graph-level representation vector.

11. The system of claim 7, wherein to obtain the graph level representation vector of the molecular graph, the one or more hardware processors are configured by the instructions to:

perform spatial graph-attention feed-forward propagation layer mechanism on the coarsened molecular graph to transform and update each of the hidden state node attributes;
perform an additional predetermined number of down-sampling and subsequent spatial graph-attention feed-forward propagation layer mechanism on the molecular graph;
perform equivalent number of up-sampling and subsequent spatial graph-attention feed-forward propagation layer mechanism to reinstate the coarsened molecular graph to an isomorphic clone of the molecular graph, wherein the isomorphic clone of the molecular graph is a node-information transformed isomorphic clone; and
feed the node-information transformed isomorphic clone of the molecular graph as an input to a node-ordering invariant read-out function to determine the graph-level representation vector.

12. The system of claim 7, wherein to obtain the graph level representation vector of the molecular graph, the one or more hardware processors are configured by the instructions to:

perform spatial Graph Attentional Propagation on the coarsened molecular graph to transform and update each of the hidden state node attributes;
perform an additional predetermined number of down-sampling and subsequent spatial Graph Attentional Propagation on the molecular graph;
perform equivalent number of up-sampling and subsequent spatial Graph Attentional Propagation to reinstate the coarsened molecular graph to an isomorphic clone of the molecular graph, wherein the isomorphic clone of the molecular graph is a node-information transformed isomorphic clone; and
feed the node-information transformed isomorphic clone of the molecular graph as an input to a node-ordering invariant read-out function to determine the graph-level representation vector.

13. One or more non-transitory machine-readable information storage mediums comprising one or more instructions which when executed by one or more hardware processors cause:

accessing a database comprising a plurality of molecular graphs associated with a plurality of molecules and a plurality of labels indicative of chemical properties of the plurality of the molecular graphs, wherein each molecular graph of the plurality of molecular graphs comprises a plurality of nodes and a plurality of edges connecting a plurality of neighboring nodes;

performing a first iteration to down-sample a molecular graph from amongst the plurality of molecular graph into a coarsened molecular graph, the first iteration comprising:

obtaining a real-valued feature matrix of a molecular graph from amongst the plurality of molecular graphs, each row vector of the real-valued feature matrix represents a feature attribute of the plurality of nodes of the molecular graph;

transforming feature attribute $\mathbf{f}_i^\ell$ associated with the node by taking a product of the feature attribute $\mathbf{f}_i^\ell$ with a feed-forward layer

$$\Gamma_\Theta(\mathbf{e}_{j,i}^{\mathcal{M}}),$$

to obtain edge-information aware node attributes

$$\mathbf{f}_i^\ell\,\Gamma_\Theta(\mathbf{e}_{j,i}^{\mathcal{M}}),$$

wherein the feed-forward layer comprises a neural network function, parameterized by 8;

computing a scalar projection $\mathbf{z_i}$ of the real-valued feature matrix $F^\ell$ on a projection vector $\mathbf{v}^\ell$, the scalar projection $\mathbf{z_i}$ comprises a projected scalar value of each node-attribute in the molecular graph on to the projection vector $\mathbf{v}^\ell$, and wherein the scalar projection $\mathbf{z_i}$ further measures a feature information of the node i to be retained when projected in the direction of learnable vector $\mathbf{v}^\ell$;

obtaining a hierarchical layer-wise propagation of a graph pooling layer of the molecular graph by taking a product of the edge-information aware node attributes

$$\mathbf{f}_i^\ell\,\Gamma_\Theta(\mathbf{e}_{j,i}^{\mathcal{M}})$$

and a unit vector associated with the projection vector $\mathbf{v}^\ell$, wherein the direction of the unit vector is same as the direction of the projection vector $\mathbf{v}^\ell$;

down-sampling the molecular graph using the hierarchical layer-wise propagation of the graph pooling layer, wherein the down-sampling of the molecular graph comprises performing a m-max-pooling operation on the molecular graph to sample a subset of m top-ranked nodes to form a coarsened molecular graph, wherein the down-sampling of the molecular graph results in rejecting a first set of nodes and retaining a second set of nodes from amongst a plurality of nodes of the molecular graph based on a ranking of the plurality of nodes, and wherein the ranking of the plurality of nodes is performed by utilizing a scalar projection scores to sample indexes of the second set of nodes;

determining a first adjacency matrix of the coarsened molecular graph using the second set of nodes; and determining a first feature matrix of the coarsened molecular graph using the second set of nodes, wherein each row of the first feature matrix corresponds to hidden state node attributes of the coarsened molecular graph;

performing one or more second iterations, wherein each of the one or more second iterations comprises performing, on the coarsened molecular graph of an immediately preceding iteration of the one or more second iterations, obtaining a real-valued feature matrix of the coarsened molecular graph, transforming feature attribute of the node, computing the scalar projection $\mathbf{z_i}$ of the real-valued feature matrix, obtaining the hierarchical layer-wise propagation of a graph pooling layer of the coarsened molecular graph, down-sampling the coarsened molecular graph using the hierarchical layer-wise propagation of the graph pooling layer, and determining a second adjacency matrix and a second feature matrix of the coarsened molecular graph; and

computing an average of the hidden state node attributes of the coarsened molecular graph obtained after preforming the one or more second iterations to obtain a graph level representation vector of the molecular graph, wherein a linear layer from the graph level representation vector assists in inductive learning tasks for molecular property prediction by reducing computational complexity.

**14.** The one or more non-transitory machine readable information storage mediums of claim 13, wherein obtaining the graph level representation vector of the molecular graph comprises:

performing spatial-graph convolution on the coarsened molecular graph to transform and update each of the hidden state node attributes;

performing an additional predetermined number of down-sampling and subsequent spatial-graph convolution on the molecular graph;

performing equivalent number of up-sampling and subsequent spatial-graph convolution to reinstate the coarsened molecular graph to an isomorphic clone of the molecular graph, wherein the isomorphic clone of the molecular graph is a node-information transformed isomorphic clone; and

feeding the node-information transformed isomorphic clone of the molecular graph as an input to a node-ordering invariant read-out function to determine the graph-level representation vector.

**15.** The one or more non-transitory machine readable information storage mediums of claim 13, wherein obtaining the graph level representation vector of the molecular graph comprises:

performing spatial dynamic neighborhood aggregation on the coarsened molecular graph to transform and update each of the hidden state node attributes;

performing an additional predetermined number of down-sampling and subsequent spatial dynamic neighborhood aggregation on the molecular graph;

performing equivalent number of up-sampling and subsequent spatial dynamic neighborhood aggregation to reinstate the coarsened molecular graph to an isomorphic clone of the molecular graph, wherein the isomorphic clone of the molecular graph is a node-information transformed isomorphic clone; and

feeding the node-information transformed isomorphic clone of the molecular graph as an input to a node-ordering invariant read-out function to determine the graph-level representation vector.

**Patentansprüche**

**1.** Prozessorimplementiertes Verfahren (300), umfassend:

Zugreifen (302), über einen oder mehrere Hardwareprozessoren, auf eine Datenbank, umfassend eine Mehrzahl von molekularen Graphen, die einer Mehrzahl von Molekülen zugeordnet sind, und eine Mehrzahl von Markierungen, die chemische Eigenschaften der Mehrzahl der molekularen Graphen anzeigen, wobei jeder molekulare Graph der Mehrzahl von molekularen Graphen eine Mehrzahl von Knoten und eine Mehrzahl von Kanten, die eine Mehrzahl von benachbarten Knoten verbinden, umfasst;

Durchführen (304), über den einen oder die mehreren Hardwareprozessoren, einer ersten Iteration zum Downsampling eines molekularen Graphen aus der Mehrzahl von molekularen Graphen in einen vergröberten molekularen Graphen, wobei die erste Iteration umfasst:

Erhalten (306) einer reellwertigen Merkmalsmatrix eines molekularen Graphen aus der Mehrzahl von molekularen Graphen, wobei jeder Zeilenvektor der reellwertigen Merkmalsmatrix ein Merkmalsattribut der Mehrzahl von Knoten des molekularen Graphen darstellt;

Transformieren (308) des Merkmalsattributs $\mathbf{f}_i^{\ell}$, das dem Knoten zugeordnet ist, durch Nehmen eines Produkts des Merkmalsattributs $\mathbf{f}_i^{\ell}$ mit einer Feed-Forward-Schicht

$$\Gamma_{\Theta}(\mathbf{e}_{j,i}^{\mathcal{M}}),$$

um kanteninformationsbewusste Knotenattribute

$$\mathbf{f}_i^{\ell}\, \Gamma_{\Theta}(\mathbf{e}_{j,i}^{\mathcal{M}})$$

zu erhalten, wobei die Feed-Forward-Schicht eine neuronale Netzwerkfunktion umfasst, parametrisiert durch $\Theta$;

Berechnen (310) einer skalaren Projektion $z_i$ der reellwertigen Merkmalsmatrix $F^\ell$ auf einen Projektionsvektor $\mathbf{v}^\ell$, wobei die skalare Projektion $z_i$ einen projizierten skalaren Wert jedes Knotenattributs in dem molekularen Graphen auf den Projektionsvektor $\mathbf{v}^\ell$ umfasst, und wobei die skalare Projektion $z_i$ ferner eine Merkmalsinformation des Knotens i misst, die beizubehalten ist, wenn sie in die Richtung des lernbaren Vektors $\mathbf{v}^\ell$ projiziert wird;

Erhalten (312) einer hierarchischen schichtweisen Ausbreitung einer Graphen-Pooling-Schicht des molekularen Graphen durch Nehmen eines Produkts der kanteninformationsbewussten Knotenattribute

$$\mathbf{f}_i^\ell \, \Gamma_\Theta(\mathbf{e}_{j,i}^{\mathcal{M}})$$

und eines Einheitsvektors, der dem Projektionsvektor $\mathbf{v}^\ell$ zugeordnet ist, wobei die Richtung des Einheitsvektors dieselbe wie die Richtung des Projektionsvektors $\mathbf{v}^\ell$; ist;

Downsampling (314) des molekularen Graphen unter Verwendung der hierarchischen schichtweisen Ausbreitung der Graphen-Pooling-Schicht, wobei das Downsampling des molekularen Graphen das Durchführen einer m-max-Pooling-Operation an dem molekularen Graphen umfasst, um einen Teilsatz von m am höchsten eingestuften Knoten abzutasten, um einen vergröberten molekularen Graphen zu bilden, wobei das Downsampling des molekularen Graphen zum Ablehnen eines ersten Satzes von Knoten und Beibehalten eines zweiten Satzes von Knoten aus einer Mehrzahl von Knoten des molekularen Graphen basierend auf einer Einstufung der Mehrzahl von Knoten führt, und wobei die Einstufung der Mehrzahl von Knoten durch Verwenden einer skalaren Projektionsbewertung durchgeführt wird, um Indizes des zweiten Satzes von Knoten abzutasten;

Bestimmen (316) einer ersten Nachbarschaftsmatrix des vergröberten molekularen Graphen unter Verwendung des zweiten Satzes von Knoten; und

Bestimmen (318) einer ersten Merkmalsmatrix des vergröberten molekularen Graphen unter Verwendung des zweiten Satzes von Knoten, wobei jede Zeile der ersten Merkmalsmatrix verborgenen Zustandsknotenattributen des vergröberten molekularen Graphen entspricht;

Durchführen (320), über den einen oder die mehreren Hardwareprozessoren, einer oder mehrerer zweiter Iterationen, wobei jede der einen oder mehreren zweiten Iterationen das Durchführen, an dem vergröberten molekularen Graphen einer unmittelbar vorhergehenden Iteration der einen oder mehreren zweiten Iterationen, des Erhaltens einer reellwertigen Merkmalsmatrix des vergröberten molekularen Graphen, des Transformierens von Merkmalsattributen des Knotens, des Berechnens der skalaren Projektion $z_i$ der reellwertigen Merkmalsmatrix, des Erhaltens der hierarchischen schichtweisen Ausbreitung einer Graphen-Pooling-Schicht des vergröberten molekularen Graphen, des Downsampling des vergröberten molekularen Graphen unter Verwendung der hierarchischen schichtweisen Ausbreitung der Graphen-Pooling-Schicht und des Bestimmens einer zweiten Nachbarschaftsmatrix und einer zweiten Merkmalsmatrix des vergröberten molekularen Graphen umfasst; und

Berechnen (322), über den einen oder die mehreren Hardwareprozessoren, eines Durchschnitts der verborgenen Zustandsknotenattribute des vergröberten molekularen Graphen, der nach dem Vorformen der einen oder mehreren zweiten Iterationen erhalten wird, um einen Graphenebenen-Darstellungsvektor des molekularen Graphen zu erhalten, wobei eine lineare Schicht aus dem Graphenebenen-Darstellungsvektor induktive Lernaufgaben zur molekularen Eigenschaftsvorhersage durch Reduzieren der Berechnungskomplexität unterstützt.

2. Prozessorimplementiertes Verfahren nach Anspruch 1, wobei das Erhalten des Graphenebenen-Darstellungsvektors des molekularen Graphen Folgendes umfasst:

Durchführen einer räumlichen Graphenfaltung an dem vergröberten molekularen Graphen, um jedes der verborgenen Zustandsknotenattribute zu transformieren und zu aktualisieren;

Durchführen einer zusätzlichen vorbestimmten Anzahl von Downsampling und anschließender räumlicher Graphenfaltung an dem molekularen Graphen;

Durchführen einer äquivalenten Anzahl von Upsampling und anschließender räumlicher Graphenfaltung, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und

Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

3. Verfahren nach Anspruch 1, wobei das Erhalten des Graphenebenen-Darstellungsvektors des molekularen Graphen Folgendes umfasst:

Durchführen einer räumlichen dynamischen Nachbarschaftsaggregation an dem vergröberten molekularen Graphen, um jedes der verborgenen Zustandsknotenattribute zu transformieren und zu aktualisieren;
Durchführen einer zusätzlichen vorbestimmten Anzahl von Downsampling und anschließender räumlicher dynamischer Nachbarschaftsaggregation an dem molekularen Graphen;
Durchführen einer äquivalenten Anzahl von Upsampling und anschließender räumlicher dynamischer Nachbarschaftsaggregation, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und
Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

4. Verfahren nach Anspruch 1, wobei das Erhalten des Graphenebenen-Darstellungsvektors des molekularen Graphen Folgendes umfasst:

Durchführen eines räumlichen Graphen-Aufmerksamkeits-Vorwärtsausbreitungsschichtmechanismus an dem vergröberten molekularen Graphen, um jedes der verborgenen Zustandsknotenattribute zu transformieren und zu aktualisieren;
Durchführen einer zusätzlichen vorbestimmten Anzahl von Downsampling und anschließendem räumlicher Graphen-Aufmerksamkeits-Vorwärtsausbreitungsschichtmechanismus an dem molekularen Graphen;
Durchführen einer äquivalenten Anzahl von Upsampling und anschließendem räumlicher Graphen-Aufmerksamkeits-Vorwärtsausbreitungsschichtmechanismus, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und
Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

5. Verfahren nach Anspruch 1, wobei das Erhalten des Graphenebenen-Darstellungsvektors des molekularen Graphen Folgendes umfasst:

Durchführen von räumlichen Identitätsabbildungsfaltungsnetzwerken an dem vergröberten molekularen Graphen, um jedes der verborgenen Zustandsknotenattribute zu transformieren und zu aktualisieren;
Durchführen einer zusätzlichen vorbestimmten Anzahl von Downsampling und anschließender räumlicher Identitätsabbildungsfaltungsnetzwerke an dem molekularen Graphen;
Durchführen einer äquivalenten Anzahl von Upsampling und anschließendem räumlichem Graphen-Aufmerksamkeits-Vorwärtsausbreitungsschichtmechanismus, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und
Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

6. Verfahren nach Anspruch 1, wobei das Erhalten des Graphenebenen-Darstellungsvektors des molekularen Graphen Folgendes umfasst:

Durchführen einer räumlichen Graphen-Aufmerksamkeitsausbreitung an dem vergröberten molekularen Graphen, um jedes der verborgenen Zustandsknotenattribute zu transformieren und zu aktualisieren;
Durchführen einer zusätzlichen vorbestimmten Anzahl von Downsampling und anschließender räumlicher Graphen-Aufmerksamkeitsausbreitung an dem molekularen Graphen;
Durchführen einer äquivalenten Anzahl von Upsampling und anschließender räumlicher Graphen-Aufmerksamkeitsausbreitung, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und

Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

7. System (500), umfassend:

einen Speicher (515), der Anweisungen speichert;

eine oder mehrere Kommunikationsschnittstellen (503); und

einen oder mehrere Hardwareprozessoren (502), die über die eine oder die mehreren Kommunikationsschnittstellen (503) mit dem Speicher (515) gekoppelt sind, wobei der eine oder die mehreren Hardwareprozessoren (502) durch die Anweisungen konfiguriert sind zum:

Zugreifen auf eine Datenbank, umfassend eine Mehrzahl von molekularen Graphen, die einer Mehrzahl von Molekülen zugeordnet sind, und eine Mehrzahl von Markierungen, die chemische Eigenschaften der Mehrzahl der molekularen Graphen anzeigen, wobei jeder molekulare Graph der Mehrzahl von molekularen Graphen eine Mehrzahl von Knoten und eine Mehrzahl von Kanten, die eine Mehrzahl von benachbarten Knoten verbinden, umfasst;

Durchführen einer ersten Iteration zum Downsampling des molekularen Graphen in einen vergröberten molekularen Graphen, wobei zum Durchführen der ersten Iteration der eine oder die mehreren Hardwareprozessoren durch die Anweisungen konfiguriert sind zum:

Erhalten einer reellwertigen Merkmalsmatrix eines molekularen Graphen aus der Mehrzahl von molekularen Graphen, wobei jeder Zeilenvektor der reellwertigen Merkmalsmatrix ein Merkmalsattribut der Mehrzahl von Knoten des molekularen Graphen darstellt;

Transformieren des Merkmalsattributs $\mathbf{f}_i^\ell$, das dem Knoten zugeordnet ist, durch Nehmen eines Produkts des Merkmalsattributs $\mathbf{f}_i^\ell$ mit einer Feed-Forward-Schicht

$$\Gamma_\Theta(\mathbf{e}_{j,i}^{\mathcal{M}}),$$

um kanteninformationsbewusste Knotenattribute

$$\mathbf{f}_i^\ell \ \Gamma_\Theta(\mathbf{e}_{j,i}^{\mathcal{M}})$$

zu erhalten, wobei die Feed-Forward-Schicht eine neuronale Netzwerkfunktion umfasst, parametrisiert durch $\Theta$;

Berechnen einer skalaren Projektion $\mathbf{z_i}$ der reellwertigen Merkmalsmatrix $F^\ell$ auf einen Projektionsvektor $\mathbf{v}^\ell$, wobei die skalare Projektion $\mathbf{z_i}$ einen projizierten skalaren Wert jedes Knotenattributs in dem molekularen Graphen auf den Projektionsvektor $\mathbf{v}^\ell$ umfasst, und wobei die skalare Projektion $\mathbf{z_i}$ ferner eine Merkmalsinformation des Knotens i misst, die beizubehalten ist, wenn sie in die Richtung des lernbaren Vektors $\mathbf{v}^\ell$ projiziert wird;

Erhalten einer hierarchischen schichtweisen Ausbreitung einer Graphen-Pooling-Schicht des molekularen Graphen durch Nehmen eines Produkts der kanteninformationsbewussten Knotenattribute

$$\mathbf{f}_i^\ell \ \Gamma_\Theta(\mathbf{e}_{j,i}^{\mathcal{M}})$$

und eines Einheitsvektors, der dem Projektionsvektor $\mathbf{v}^\ell$, zugeordnet ist, wobei die Richtung des Einheitsvektors dieselbe wie die Richtung des Projektionsvektors $\mathbf{v}^\ell$; ist;

Downsampling des molekularen Graphen unter Verwendung der hierarchischen schichtweisen Ausbreitung der Graphen-Pooling-Schicht, wobei das Downsampling des molekularen Graphen das Durchführen einer m-max-Pooling-Operation an dem molekularen Graphen umfasst, um einen Teilsatz von m am höchsten eingestuften Knoten abzutasten, um einen vergröberten molekularen Graphen zu bilden, wobei das Downsampling des molekularen Graphen zum Ablehnen eines ersten Satzes von Knoten und Beibehalten eines zweiten Satzes von Knoten aus einer Mehrzahl von Knoten des molekularen Graphen basierend auf einer Einstufung der Mehrzahl von Knoten führt, und wobei die Einstufung der Mehrzahl von Knoten durch Verwenden einer skalaren Projektionsbewertung durchgeführt wird, um Indizes des zweiten Satzes von Knoten abzutasten;

Bestimmen einer ersten Nachbarschaftsmatrix des vergröberten molekularen Graphen unter Verwendung des zweiten Satzes von Knoten;

Bestimmen einer ersten Merkmalsmatrix des vergröberten molekularen Graphen unter Verwendung des zweiten Satzes von Knoten, wobei jede Zeile der ersten Merkmalsmatrix verborgenen Zustandsknotenattributen des vergröberten molekularen Graphen entspricht;

Durchführen einer oder mehrerer zweiter Iterationen, wobei jede der einen oder mehreren zweiten Iterationen das Durchführen, an dem vergröberten molekularen Graphen einer unmittelbar vorhergehenden Iteration der einen oder mehreren zweiten Iterationen, des Erhaltens einer reellwertigen Merkmalsmatrix des vergröberten molekularen Graphen, des Transformierens von Merkmalsattributen des Knotens, des Berechnens der skalaren Projektion $z_i$ der reellwertigen Merkmalsmatrix, des Erhaltens der hierarchischen schichtweisen Ausbreitung einer Graphen-Pooling-Schicht des vergröberten molekularen Graphen, des Downsampling des vergröberten molekularen Graphen unter Verwendung der hierarchischen schichtweisen Ausbreitung der Graphen-Pooling-Schicht und des Bestimmens einer zweiten Nachbarschaftsmatrix und einer zweiten Merkmalsmatrix des vergröberten molekularen Graphen umfasst; und

Berechnen eines Durchschnitts der verborgenen Zustandsknotenattribute des vergröberten molekularen Graphen, der nach dem Vorformen der einen oder mehreren zweiten Iterationen erhalten wird, um einen Graphenebenen-Darstellungsvektor des molekularen Graphen zu erhalten, wobei eine lineare Schicht aus dem Graphenebenen-Darstellungsvektor induktive Lernaufgaben zur molekularen Eigenschaftsvorhersage durch Reduzieren der Berechnungskomplexität unterstützt.

8. System nach Anspruch 7, wobei zum Erhalten des Graphenebenen-Darstellungsvektors des molekularen Graphen der eine oder die mehreren Hardwareprozessoren durch die Anweisungen konfiguriert sind zum:

Durchführen einer räumlichen Graphenfaltung an dem vergröberten molekularen Graphen, um jedes der verborgenen Zustandsknotenattribute zu transformieren und zu aktualisieren;

Durchführen einer zusätzlichen vorbestimmten Anzahl von Downsampling und anschließender räumlicher Graphenfaltung an dem molekularen Graphen;

Durchführen einer äquivalenten Anzahl von Upsampling und anschließender räumlicher Graphenfaltung, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und

Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

9. System nach Anspruch 7, wobei zum Erhalten des Graphenebenen-Darstellungsvektors des molekularen Graphen der eine oder die mehreren Hardwareprozessoren durch die Anweisungen konfiguriert sind zum:

Durchführen einer räumlichen dynamischen Nachbarschaftsaggregation an dem vergröberten molekularen Graphen, um jedes der verborgenen Zustandsknotenattribute zu transformieren und zu aktualisieren;

Durchführen einer zusätzlichen vorbestimmten Anzahl von Downsampling und anschließender räumlicher dynamischer Nachbarschaftsaggregation an dem molekularen Graphen;

Durchführen einer äquivalenten Anzahl von Upsampling und anschließender räumlicher dynamischer Nachbarschaftsaggregation, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und

Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

10. System nach Anspruch 7, wobei zum Erhalten des Graphenebenen-Darstellungsvektors des molekularen Graphen der eine oder die mehreren Hardwareprozessoren durch die Anweisungen konfiguriert sind zum:

Durchführen eines räumlichen Graphen-Aufmerksamkeits-Vorwärtsausbreitungsschichtmechanismus an dem vergröberten molekularen Graphen, um jedes der verborgenen Zustandsknotenattribute zu transformieren und zu aktualisieren;

Durchführen einer zusätzlichen vorbestimmten Anzahl von Downsampling und anschließendem räumlichem Graphen-Aufmerksamkeits-Vorwärtsausbreitungsschichtmechanismus an dem molekularen Graphen;

Durchführen einer äquivalenten Anzahl von Upsampling und anschließendem räumlichem Graphen-Aufmerksamkeits-Vorwärtsausbreitungsschichtmechanismus, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und

Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

11. System nach Anspruch 7, wobei zum Erhalten des Graphenebenen-Darstellungsvektors des molekularen Graphen der eine oder die mehreren Hardwareprozessoren durch die Anweisungen konfiguriert sind zum:

Durchführen eines räumlichen Graphen-Aufmerksamkeits-Vorwärtsausbreitungsschichtmechanismus an dem vergröberten molekularen Graphen, um jedes der verborgenen Zustandsknotenattribute zu transformieren und zu aktualisieren;

Durchführen einer zusätzlichen vorbestimmten Anzahl von Downsampling und anschließendem räumlichem Graphen-Aufmerksamkeits-Vorwärtsausbreitungsschichtmechanismus an dem molekularen Graphen;

Durchführen einer äquivalenten Anzahl von Upsampling und anschließendem räumlichem Graphen-Aufmerksamkeits-Vorwärtsausbreitungsschichtmechanismus, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und

Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

12. System nach Anspruch 7, wobei zum Erhalten des Graphenebenen-Darstellungsvektors des molekularen Graphen der eine oder die mehreren Hardwareprozessoren durch die Anweisungen konfiguriert sind zum:

Durchführen einer räumlichen Graphen-Aufmerksamkeitsausbreitung an dem vergröberten molekularen Graphen, um jedes der verborgenen Zustandsknotenattribute zu transformieren und zu aktualisieren;

Durchführen einer zusätzlichen vorbestimmten Anzahl von Downsampling und anschließender räumlicher Graphen-Aufmerksamkeitsausbreitung an dem molekularen Graphen;

Durchführen einer äquivalenten Anzahl von Upsampling und anschließender räumlicher Graphen-Aufmerksamkeitsausbreitung, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und

Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

13. Ein oder mehrere nichtflüchtige maschinenlesbare Informationsspeichermedien, umfassend eine oder mehrere Anweisungen, die bei Ausführung durch einen oder mehrere Hardwareprozessoren bewirken:

Zugreifen auf eine Datenbank, umfassend eine Mehrzahl von molekularen Graphen, die einer Mehrzahl von Molekülen zugeordnet sind, und eine Mehrzahl von Markierungen, die chemische Eigenschaften der Mehrzahl der molekularen Graphen anzeigen, wobei jeder molekulare Graph der Mehrzahl von molekularen Graphen eine Mehrzahl von Knoten und eine Mehrzahl von Kanten, die eine Mehrzahl von benachbarten Knoten verbinden, umfasst;

Durchführen einer ersten Iteration zum Downsampling eines molekularen Graphen aus der Mehrzahl von molekularen Graphen in einen vergröberten molekularen Graphen, wobei die erste Iteration umfasst:

Erhalten einer reellwertigen Merkmalsmatrix eines molekularen Graphen aus der Mehrzahl von molekularen Graphen, wobei jeder Zeilenvektor der reellwertigen Merkmalsmatrix ein Merkmalsattribut der Mehrzahl von Knoten des molekularen Graphen darstellt;

Transformieren des Merkmalsattributs $\mathbf{f}_i^\ell$, das dem Knoten zugeordnet ist, durch Nehmen eines Produkts des Merkmalsattributs $\mathbf{f}_i^\ell$ mit einer Feed-Forward-Schicht

$$\Gamma_{\Theta}(\mathbf{e}_{j,i}^{\mathcal{M}}),$$

um kanteninformationsbewusste Knotenattribute

$$\mathbf{f}_i^\ell \, \Gamma_{\Theta}(\mathbf{e}_{j,i}^{\mathcal{M}})$$

zu erhalten, wobei die Feed-Forward-Schicht eine neuronale Netzwerkfunktion umfasst, parametrisiert durch $\Theta$;

Berechnen einer skalaren Projektion $\mathbf{z_i}$ der reellwertigen Merkmalsmatrix $F^\ell$ auf einen Projektionsvektor $\mathbf{v}^\ell$, wobei die skalare Projektion $\mathbf{z_i}$ einen projizierten skalaren Wert jedes Knotenattributs in dem molekularen Graphen auf den Projektionsvektor $\mathbf{v}^\ell$ umfasst, und wobei die skalare Projektion $\mathbf{z_i}$ ferner eine Merkmals-information des Knotens i misst, die beizubehalten ist, wenn sie in die Richtung des lernbaren Vektors $\mathbf{v}^\ell$ projiziert wird;

Erhalten einer hierarchischen schichtweisen Ausbreitung einer Graphen-Pooling-Schicht des molekularen Graphen durch Nehmen eines Produkts der kanteninformationsbewussten Knotenattribute

$$\mathbf{f}_i^\ell \, \Gamma_{\Theta}(\mathbf{e}_{j,i}^{\mathcal{M}})$$

und eines Einheitsvektors, der dem Projektionsvektor $\mathbf{v}^\ell$ zugeordnet ist, wobei die Richtung des Einheitsvektors dieselbe wie die Richtung des Projektionsvektors $\mathbf{v}^\ell$; ist;

Downsampling des molekularen Graphen unter Verwendung der hierarchischen schichtweisen Ausbreitung der Graphen-Pooling-Schicht, wobei das Downsampling des molekularen Graphen das Durchführen einer m-max-Pooling-Operation an dem molekularen Graphen umfasst, um einen Teilsatz von m am höchsten eingestuften Knoten abzutasten, um einen vergröberten molekularen Graphen zu bilden, wobei das Downsampling des molekularen Graphen zum Ablehnen eines ersten Satzes von Knoten und Beibehalten eines zweiten Satzes von Knoten aus einer Mehrzahl von Knoten des molekularen Graphen basierend auf einer Einstufung der Mehrzahl von Knoten führt, und wobei die Einstufung der Mehrzahl von Knoten durch Verwenden einer skalaren Projektionsbewertung durchgeführt wird, um Indizes des zweiten Satzes von Knoten abzutasten;

Bestimmen einer ersten Nachbarschaftsmatrix des vergröberten molekularen Graphen unter Verwendung des zweiten Satzes von Knoten; und

Bestimmen einer ersten Merkmalsmatrix des vergröberten molekularen Graphen unter Verwendung des zweiten Satzes von Knoten, wobei jede Zeile der ersten Merkmalsmatrix verborgenen Zustandsknotenattributen des vergröberten molekularen Graphen entspricht;

Durchführen einer oder mehrerer zweiter Iterationen, wobei jede der einen oder mehreren zweiten Iterationen das Durchführen, an dem vergröberten molekularen Graphen einer unmittelbar vorhergehenden Iteration der einen oder mehreren zweiten Iterationen, des Erhaltens einer reellwertigen Merkmalsmatrix des vergröberten molekularen Graphen, des Transformierens von Merkmalsattributen des Knotens, des Berechnens der skalaren Projektion $\mathbf{z_i}$ der reellwertigen Merkmalsmatrix, des Erhaltens der hierarchischen schichtweisen Ausbreitung einer Graphen-Pooling-Schicht des vergröberten molekularen Graphen, des Downsampling des vergröberten molekularen Graphen unter Verwendung der hierarchischen schichtweisen Ausbreitung der Graphen-Pooling-Schicht und des Bestimmens einer zweiten Nachbarschaftsmatrix und einer zweiten Merkmalsmatrix des vergröberten molekularen Graphen umfasst; und

Berechnen eines Durchschnitts der verborgenen Zustandsknotenattribute des vergröberten molekularen Graphen, der nach dem Vorformen der einen oder mehreren zweiten Iterationen erhalten wird, um einen Graphenebenen-Darstellungsvektor des molekularen Graphen zu erhalten, wobei eine lineare Schicht aus dem Graphenebenen-Darstellungsvektor induktive Lernaufgaben zur molekularen Eigenschaftsvorhersage durch Reduzieren der Berechnungskomplexität unterstützt.

14. Ein oder mehrere nichtflüchtige maschinenlesbare Informationsspeichermedien nach Anspruch 13, wobei das Erhalten des Graphenebenen-Darstellungsvektors des molekularen Graphen Folgendes umfasst:

Durchführen einer räumlichen Graphenfaltung an dem vergröberten molekularen Graphen, um jedes der verborgenen Zustandsknotenattribute zu transformieren und zu aktualisieren;
Durchführen einer zusätzlichen vorbestimmten Anzahl von Downsampling und anschließender räumlicher Graphenfaltung an dem molekularen Graphen;
Durchführen einer äquivalenten Anzahl von Upsampling und anschließender räumlicher Graphenfaltung, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und
Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

15. Ein oder mehrere nichtflüchtige maschinenlesbare Informationsspeichermedien nach Anspruch 13, wobei das Erhalten des Graphenebenen-Darstellungsvektors des molekularen Graphen Folgendes umfasst:

Durchführen einer räumlichen dynamischen Nachbarschaftsaggregation an dem vergröberten molekularen Graphen, um jedes der verborgenen Zustandsknotenattribute zu transformieren und zu aktualisieren;
Durchführen einer zusätzlichen vorbestimmten Anzahl von Downsampling und anschließender räumlicher dynamischer Nachbarschaftsaggregation an dem molekularen Graphen;
Durchführen einer äquivalenten Anzahl von Upsampling und anschließender räumlicher dynamischer Nachbarschaftsaggregation, um den vergröberten molekularen Graphen in einen isomorphen Klon des molekularen Graphen wiederherzustellen, wobei der isomorphe Klon des molekularen Graphen ein knoteninformationstransformierter isomorpher Klon ist; und
Einspeisen des knoteninformationstransformierten isomorphen Klons des molekularen Graphen als eine Eingabe in eine knotenordnungsinvariante Auslesefunktion, um den Graphenebenen-Darstellungsvektor zu bestimmen.

## Revendications

1. Procédé mis en œuvre par processeur (300), comprenant :

l'accès (302), via un ou plusieurs processeurs matériels, à une base de données comprenant une pluralité de graphes moléculaires associés à une pluralité de molécules et une pluralité d'étiquettes indicatives de propriétés chimiques de la pluralité des graphes moléculaires, dans lequel chaque graphe moléculaire de la pluralité de graphes moléculaires comprend une pluralité de nœuds et une pluralité de bords reliant une pluralité de nœuds voisins ;
la réalisation (304), via les un ou plusieurs processeurs matériels, d'une première itération pour sous-échantillonner un graphe moléculaire parmi la pluralité de graphes moléculaires en un graphe moléculaire affiné, la première itération comprenant :

l'obtention (306) d'une matrice de caractéristiques à valeur réelle d'un graphe moléculaire parmi la pluralité de graphes moléculaires, chaque vecteur de rangée de la matrice de caractéristiques à valeur réelle représentant un attribut de caractéristique de la pluralité de nœuds du graphe moléculaire ;

la transformation (308) de l'attribut de caractéristique $\mathbf{f}_i^\ell$ associé au nœud en prenant un produit de l'attribut de caractéristique $\mathbf{f}_i^\ell$ avec une couche d'action directe

$$\Gamma_\Theta(\mathbf{e}_{j,i}^{\mathcal{M}}),$$

pour obtenir des attributs de nœud sensibles aux informations de bord

$$\mathbf{f}_i^{\ell} \ \Gamma_{\Theta}(\mathbf{e}_{j,i}^{\mathcal{M}}),$$

, dans lequel la couche d'action directe comprend une fonction de réseau neuronal, paramétrée par $\Theta$ ;

le calcul (310) d'une projection scalaire $\mathbf{z_i}$ de la matrice de caractéristiques à valeur réelle $F^{\ell}$ sur un vecteur de projection $\mathbf{v}^{\ell}$, la projection scalaire $\mathbf{z_i}$ comprenant une valeur scalaire projetée de chaque attribut de nœud dans le graphe moléculaire sur le vecteur de projection $\mathbf{v}^{\ell}$, et dans lequel la projection scalaire $\mathbf{z_i}$ mesure en outre des informations de caractéristique du nœud i à retenir lorsqu'elles sont projetées dans la direction du vecteur de projection $\mathbf{v}^{\ell}$ ;

l'obtention (312) d'une propagation hiérarchique par couche d'une couche de regroupement de graphes du graphe moléculaire en prenant un produit des attributs de nœud sensibles aux informations de bord

$$\mathbf{f}_i^{\ell} \ \Gamma_{\Theta}(\mathbf{e}_{j,i}^{\mathcal{M}})$$

et d'un vecteur unitaire associé au vecteur de projection $\mathbf{v}^{\ell}$, dans lequel la direction du vecteur unitaire est la même que la direction du vecteur de projection $\mathbf{v}^{\ell}$ ;

le sous-échantillonnage (314) du graphe moléculaire en utilisant la propagation hiérarchique par couche de la couche de regroupement de graphes, dans lequel le sous-échantillonnage du graphe moléculaire comprend la réalisation d'une opération de regroupement m-max sur le graphe moléculaire pour échantillonner un sous-ensemble de m nœuds de rang supérieur pour former un graphe moléculaire affiné, dans lequel le sous-échantillonnage du graphe moléculaire a pour résultat le rejet d'un premier ensemble de nœuds et la retenue d'un second ensemble de nœuds parmi une pluralité de nœuds du graphe moléculaire sur la base d'un classement de la pluralité de nœuds, et dans lequel le classement de la pluralité de nœuds est réalisé en utilisant des scores de projection scalaire pour échantillonner des indices du second ensemble de nœuds ;

la détermination (316) d'une première matrice d'adjacence du graphe moléculaire affiné en utilisant le second ensemble de nœuds ; et

la détermination (318) d'une première matrice de caractéristiques du graphe moléculaire affiné en utilisant le second ensemble de nœuds, dans lequel chaque rangée de la première matrice de caractéristiques correspond à des attributs de nœud d'état caché du graphe moléculaire affiné ;

la réalisation (320), via les un ou plusieurs processeurs matériels, d'une ou plusieurs secondes itérations, dans lequel chacune des une ou plusieurs secondes itérations comprend la réalisation, sur le graphe moléculaire affiné d'une itération immédiatement précédente des une ou plusieurs secondes itérations, de l'obtention d'une matrice de caractéristiques à valeur réelle du graphe moléculaire affiné, de la transformation d'attributs de caractéristiques du nœud, du calcul de la projection scalaire $\mathbf{z_i}$ de la matrice de caractéristiques à valeur réelle, de l'obtention de la propagation hiérarchique par couche d'une couche de regroupement de graphes du graphe moléculaire affiné, du sous-échantillonnage du graphe moléculaire affiné en utilisant la propagation hiérarchique par couche de la couche de regroupement de graphes, et de la détermination d'une seconde matrice d'adjacence et d'une seconde matrice de caractéristiques du graphe moléculaire affiné ; et

le calcul (322), via les un ou plusieurs processeurs matériels, d'une moyenne des attributs de nœud d'état caché du graphe moléculaire affiné obtenus après la préformation des une ou plusieurs secondes itérations pour obtenir un vecteur de représentation de niveau de graphe du graphe moléculaire, dans lequel une couche linéaire à partir du vecteur de représentation de niveau de graphe aide à des tâches d'apprentissage inductif pour une prédiction de propriété moléculaire en réduisant la complexité de calcul.

2. Procédé mis en œuvre par processeur selon la revendication 1, dans lequel l'obtention du vecteur de représentation de niveau de graphe du graphe moléculaire comprend :

la réalisation d'une convolution de graphe spatial sur le graphe moléculaire affiné pour transformer et mettre à jour chacun des attributs de nœud d'état caché ;

la réalisation d'un nombre prédéterminé supplémentaire de sous-échantillonnage et de convolution de graphe spatial subséquente sur le graphe moléculaire ;

la réalisation d'un nombre équivalent de sur-échantillonnage et de convolution de graphe spatial subséquente pour rétablir le graphe moléculaire affiné en un clone isomorphe du graphe moléculaire, dans lequel le clone isomorphe du graphe moléculaire est un clone isomorphe transformé par des informations de nœud ; et l'alimentation du clone isomorphe transformé par des informations de nœud du graphe moléculaire en tant qu'entrée à une fonction de lecture invariante d'ordre de nœud pour déterminer le vecteur de représentation de niveau de graphe.

3. Procédé selon la revendication 1, dans lequel l'obtention du vecteur de représentation de niveau de graphe du graphe moléculaire comprend :

la réalisation d'une agrégation de voisinage dynamique spatial sur le graphe moléculaire affiné pour transformer et mettre à jour chacun des attributs de nœud d'état caché ;
la réalisation d'un nombre prédéterminé supplémentaire d'étapes de sous-échantillonnage et d'agrégation de voisinage dynamique spatial subséquente sur le graphe moléculaire ;
la réalisation d'un nombre équivalent de sur-échantillonnage et d'agrégation de voisinage dynamique spatial subséquente pour rétablir le graphe moléculaire affiné en un clone isomorphe du graphe moléculaire, dans lequel le clone isomorphe du graphe moléculaire est un clone isomorphe transformé par des informations de nœud ; et
l'alimentation du clone isomorphe transformé par des informations de nœud du graphe moléculaire en tant qu'entrée à une fonction de lecture invariante d'ordre de nœud pour déterminer le vecteur de représentation de niveau de graphe.

4. Procédé selon la revendication 1, dans lequel l'obtention du vecteur de représentation de niveau de graphe du graphe moléculaire comprend :

la réalisation d'un mécanisme de couche de propagation à action directe d'attention de graphe spatial sur le graphe moléculaire affiné pour transformer et mettre à jour chacun des attributs de nœud d'état caché ;
la réalisation d'un nombre prédéterminé supplémentaire de sous-échantillonnage et de mécanisme de couche de propagation à action directe d'attention de graphe spatial subséquent sur le graphe moléculaire ;
la réalisation d'un nombre équivalent de sur-échantillonnage et de mécanisme de couche de propagation à action directe d'attention de graphe spatial subséquent pour rétablir le graphe moléculaire affiné en un clone isomorphe du graphe moléculaire, dans lequel le clone isomorphe du graphe moléculaire est un clone isomorphe transformé par des informations de nœud ; et
l'alimentation du clone isomorphe transformé par des informations de nœud du graphe moléculaire en tant qu'entrée à une fonction de lecture invariante d'ordre de nœud pour déterminer le vecteur de représentation de niveau de graphe.

5. Procédé selon la revendication 1, dans lequel l'obtention du vecteur de représentation de niveau de graphe du graphe moléculaire comprend :

la réalisation de réseaux de convolution de mappage d'identité spatiale sur le graphe moléculaire affiné pour transformer et mettre à jour chacun des attributs de nœud d'état caché ;
la réalisation d'un nombre prédéterminé supplémentaire de sous-échantillonnage et de réseaux de convolution de mappage d'identité spatiale subséquente sur le graphe moléculaire ;
la réalisation d'un nombre équivalent de sur-échantillonnage et de mécanisme de couche de propagation à action directe d'attention de graphe spatial subséquente pour rétablir le graphe moléculaire affiné en un clone isomorphe du graphe moléculaire, dans lequel le clone isomorphe du graphe moléculaire est un clone isomorphe transformé par des informations de nœud ; et
l'alimentation du clone isomorphe transformé par des informations de nœud du graphe moléculaire en tant qu'entrée à une fonction de lecture invariante d'ordre de nœud pour déterminer le vecteur de représentation de niveau de graphe.

6. Procédé selon la revendication 1, dans lequel l'obtention du vecteur de représentation de niveau de graphe du graphe moléculaire comprend :

la réalisation d'une propagation d'attention de graphe spatial sur le graphe moléculaire affiné pour transformer et mettre à jour chacun des attributs de nœud d'état caché ;
la réalisation d'un nombre prédéterminé supplémentaire de sous-échantillonnage et de propagation d'attention de graphe spatial subséquente sur le graphe moléculaire ;
la réalisation d'un nombre équivalent de sur-échantillonnage et de propagation d'attention de graphe spatial subséquente pour rétablir le graphe moléculaire affiné en un clone isomorphe du graphe moléculaire, dans lequel le clone isomorphe du graphe moléculaire est un clone isomorphe transformé par des informations de nœud ; et l'alimentation du clone isomorphe transformé par des informations de nœud du graphe moléculaire en tant qu'entrée à une fonction de lecture invariante d'ordre de nœud pour déterminer le vecteur de représentation de niveau de graphe.

**7.** Système (500), comprenant :

une mémoire (515) stockant des instructions ;
une ou plusieurs interfaces de communication (503) ; et
un ou plusieurs processeurs matériels (502) couplés à la mémoire (515) via les une ou plusieurs interfaces de communication (503), dans lequel les un ou plusieurs processeurs matériels (502) sont configurés par les instructions pour :

accéder à une base de données comprenant une pluralité de graphes moléculaires associés à une pluralité de molécules et une pluralité d'étiquettes indicatives de propriétés chimiques de la pluralité des graphes moléculaires, dans lequel chaque graphe moléculaire de la pluralité de graphes moléculaires comprend une pluralité de nœuds et une pluralité de bords reliant une pluralité de nœuds voisins ;
réaliser une première itération pour sous-échantillonner le graphe moléculaire en un graphe moléculaire affiné, dans lequel pour réaliser la première itération, les un ou plusieurs processeurs matériels sont configurés par les instructions pour :

obtenir une matrice de caractéristiques à valeur réelle d'un graphe moléculaire parmi la pluralité de graphes moléculaires, chaque vecteur de rangée de la matrice de caractéristiques à valeur réelle représentant un attribut de caractéristique de la pluralité de nœuds du graphe moléculaire ;

transformer l'attribut de caractéristique $\mathbf{f}_i^\ell$ associé au nœud en prenant un produit de l'attribut de caractéristique $\mathbf{f}_i^\ell$ avec une couche d'action directe

$$\Gamma_\Theta(\mathbf{e}_{j,i}^{\mathcal{M}}),$$

pour obtenir des attributs de nœud sensibles aux informations de bord

$$\mathbf{f}_i^\ell \, \Gamma_\Theta(\mathbf{e}_{j,i}^{\mathcal{M}}),$$

, dans lequel la couche d'action directe comprend une fonction de réseau neuronal, paramétrée par $\Theta$ ;
calculer une projection scalaire $\mathbf{z_i}$ de la matrice de caractéristiques à valeur réelle $F^\ell$ sur un vecteur de projection $\mathbf{v}^\ell$, la projection scalaire $\mathbf{z_i}$ comprenant une valeur scalaire projetée de chaque attribut de nœud dans le graphe moléculaire sur le vecteur de projection $\mathbf{v}^\ell$, et dans lequel la projection scalaire $\mathbf{z_i}$ mesure en outre des informations de caractéristique du nœud $i$ à retenir lorsqu'elles sont projetées dans la direction du vecteur de projection $\mathbf{v}^\ell$ ;
obtenir une propagation hiérarchique par couche d'une couche de regroupement de graphes du graphe moléculaire en prenant un produit des attributs de nœud sensibles aux informations de bord

$$\mathbf{f}_i^\ell \, \Gamma_\Theta(\mathbf{e}_{j,i}^{\mathcal{M}})$$

et d'un vecteur unitaire associé au vecteur de projection $\mathbf{v}^\ell$,, dans lequel la direction du vecteur unitaire est la même que la direction du vecteur de projection $\mathbf{v}^\ell$;
sous-échantillonner le graphe moléculaire en utilisant la propagation hiérarchique par couche de la couche de regroupement de graphes, dans lequel le sous-échantillonnage du graphe moléculaire comprend la réalisation d'une opération de regroupement m-max sur le graphe moléculaire pour échantillonner un sous-ensemble de m nœuds de rang supérieur pour former un graphe moléculaire affiné, dans lequel le sous-échantillonnage du graphe moléculaire a pour résultat le rejet d'un premier ensemble de nœuds et la retenue d'un second ensemble de nœuds parmi une pluralité de nœuds du graphe moléculaire sur la base d'un classement de la pluralité de nœuds, et dans lequel le classement de la pluralité de nœuds est réalisé en utilisant des scores de projection scalaire pour échantillonner des indices du second ensemble de nœuds ;

déterminer une première matrice d'adjacence du graphe moléculaire affiné en utilisant le second ensemble de nœuds ;

déterminer une première matrice de caractéristiques du graphe moléculaire affiné en utilisant le second ensemble de nœuds, dans lequel chaque rangée de la première matrice de caractéristiques correspond à des attributs de nœud d'état caché du graphe moléculaire affiné ;

réaliser une ou plusieurs secondes itérations, dans lequel chacune des une ou plusieurs secondes itérations comprend la réalisation, sur le graphe moléculaire affiné d'une itération immédiatement précédente des une ou plusieurs secondes itérations, de l'obtention d'une matrice de caractéristiques à valeur réelle du graphe moléculaire affiné, de la transformation d'attributs de caractéristiques du nœud, du calcul de la projection scalaire $z_i$ de la matrice de caractéristiques à valeur réelle, de l'obtention de la propagation hiérarchique par couche d'une couche de regroupement de graphes du graphe moléculaire affiné, du sous-échantillonnage du graphe moléculaire affiné en utilisant la propagation hiérarchique par couche de la couche de regroupement de graphes, et de la détermination d'une seconde matrice d'adjacence et d'une seconde matrice de caractéristiques du graphe moléculaire affiné ; et

calculer une moyenne des attributs de nœud d'état caché du graphe moléculaire affiné obtenus après la préformation des une ou plusieurs secondes itérations pour obtenir un vecteur de représentation de niveau de graphe du graphe moléculaire, dans lequel une couche linéaire à partir du vecteur de représentation de niveau de graphe aide à des tâches d'apprentissage inductif pour une prédiction de propriété moléculaire en réduisant la complexité de calcul.

8. Système selon la revendication 7, dans lequel pour obtenir le vecteur de représentation de niveau de graphe du graphe moléculaire, les un ou plusieurs processeurs matériels sont configurés par les instructions pour :

réaliser une convolution de graphe spatial sur le graphe moléculaire affiné pour transformer et mettre à jour chacun des attributs de nœud d'état caché ;

réaliser un nombre prédéterminé supplémentaire de sous-échantillonnage et de convolution de graphe spatial subséquente sur le graphe moléculaire ;

réaliser un nombre équivalent de sur-échantillonnage et de convolution de graphe spatial subséquente pour rétablir le graphe moléculaire affiné en un clone isomorphe du graphe moléculaire, dans lequel le clone isomorphe du graphe moléculaire est un clone isomorphe transformé par des informations de nœud ; et

alimenter le clone isomorphe transformé par des informations de nœud du graphe moléculaire en tant qu'entrée à une fonction de lecture invariante d'ordre de nœud pour déterminer le vecteur de représentation de niveau de graphe.

9. Système selon la revendication 7, dans lequel pour obtenir le vecteur de représentation de niveau de graphe du graphe moléculaire, les un ou plusieurs processeurs matériels sont configurés par les instructions pour :

réaliser une agrégation de voisinage dynamique spatial sur le graphe moléculaire affiné pour transformer et mettre à jour chacun des attributs de nœud d'état caché ;

réaliser un nombre prédéterminé supplémentaire de sous-échantillonnage et d'agrégation de voisinage dynamique spatial subséquente sur le graphe moléculaire ;

réaliser un nombre équivalent de sur-échantillonnage et d'agrégation de voisinage dynamique spatial subséquente pour rétablir le graphe moléculaire affiné en un clone isomorphe du graphe moléculaire, dans lequel le clone isomorphe du graphe moléculaire est un clone isomorphe transformé par des informations de nœud ; et

alimenter le clone isomorphe transformé par des informations de nœud du graphe moléculaire en tant qu'entrée à une fonction de lecture invariante d'ordre de nœud pour déterminer le vecteur de représentation de niveau de graphe.

10. Système selon la revendication 7, dans lequel pour obtenir le vecteur de représentation de niveau de graphe du graphe moléculaire, les un ou plusieurs processeurs matériels sont configurés par les instructions pour :

réaliser un mécanisme de couche de propagation à action directe d'attention de graphe spatial sur le graphe moléculaire affiné pour transformer et mettre à jour chacun des attributs de nœud d'état caché ;

réaliser un nombre prédéterminé supplémentaire de sous-échantillonnage et de mécanisme de couche de propagation à action directe d'attention de graphe spatial subséquent sur le graphe moléculaire ;

réaliser un nombre équivalent de sur-échantillonnage et de mécanisme de couche de propagation à action directe d'attention de graphe spatial subséquent pour rétablir le graphe moléculaire affiné en un clone isomorphe du graphe moléculaire, dans lequel le clone isomorphe du graphe moléculaire est un clone isomorphe transformé par des informations de nœud ; et

alimenter le clone isomorphe transformé par des informations de nœud du graphe moléculaire en tant qu'entrée à une fonction de lecture invariante d'ordre de nœud pour déterminer le vecteur de représentation de niveau de graphe.

**11.** Système selon la revendication 7, dans lequel pour obtenir le vecteur de représentation de niveau de graphe du graphe moléculaire, les un ou plusieurs processeurs matériels sont configurés par les instructions pour :

réaliser un mécanisme de couche de propagation à action directe d'attention de graphe spatial sur le graphe moléculaire affiné pour transformer et mettre à jour chacun des attributs de nœud d'état caché ;

réaliser un nombre prédéterminé supplémentaire de sous-échantillonnage et de mécanisme de couche de propagation à action directe d'attention de graphe spatial subséquent sur le graphe moléculaire ;

réaliser un nombre équivalent de sur-échantillonnage et de mécanisme de couche de propagation à action directe d'attention de graphe spatial subséquent pour rétablir le graphe moléculaire affiné en un clone isomorphe du graphe moléculaire, dans lequel le clone isomorphe du graphe moléculaire est un clone isomorphe transformé par des informations de nœud ; et

alimenter le clone isomorphe transformé par des informations de nœud du graphe moléculaire en tant qu'entrée à une fonction de lecture invariante d'ordre de nœud pour déterminer le vecteur de représentation de niveau de graphe.

**12.** Système selon la revendication 7, dans lequel pour obtenir le vecteur de représentation de niveau de graphe du graphe moléculaire, les un ou plusieurs processeurs matériels sont configurés par les instructions pour :

réaliser une propagation d'attention de graphe spatial sur le graphe moléculaire affiné pour transformer et mettre à jour chacun des attributs de nœud d'état caché ;

réaliser un nombre prédéterminé supplémentaire de sous-échantillonnage et de propagation d'attention de graphe spatial subséquente sur le graphe moléculaire ;

réaliser un nombre équivalent de sur-échantillonnage et de propagation d'attention de graphe spatial subséquente pour rétablir le graphe moléculaire affiné en un clone isomorphe du graphe moléculaire, dans lequel le clone isomorphe du graphe moléculaire est un clone isomorphe transformé par des informations de nœud ; et

alimenter le clone isomorphe transformé par des informations de nœud du graphe moléculaire en tant qu'entrée à une fonction de lecture invariante d'ordre de nœud pour déterminer le vecteur de représentation de niveau de graphe.

**13.** Un ou plusieurs supports de stockage d'informations lisibles par machine non transitoires comprenant une ou plusieurs instructions qui, lorsqu'elles sont exécutées par un ou plusieurs processeurs matériels, amènent :

l'accès à une base de données comprenant une pluralité de graphes moléculaires associés à une pluralité de molécules et une pluralité d'étiquettes indicatives de propriétés chimiques de la pluralité des graphes moléculaires, dans lequel chaque graphe moléculaire de la pluralité de graphes moléculaires comprend une pluralité de nœuds et une pluralité de bords reliant une pluralité de nœuds voisins ;

la réalisation d'une première itération pour sous-échantillonner un graphe moléculaire parmi la pluralité de graphes moléculaires en un graphe moléculaire affiné, la première itération comprenant :

l'obtention d'une matrice de caractéristiques à valeur réelle d'un graphe moléculaire parmi la pluralité de graphes moléculaires, chaque vecteur de rangée de la matrice de caractéristiques à valeur réelle représentant un attribut de caractéristique de la pluralité de nœuds du graphe moléculaire ;

la transformation de l'attribut de caractéristique $\mathbf{f}_i^{\ell}$ associé au nœud en prenant un produit de l'attribut de caractéristique $\mathbf{f}_i^{\ell}$ avec une couche d'action directe

$$\Gamma_{\Theta}(\mathbf{e}_{j,i}^{\mathcal{M}}),$$

pour obtenir des attributs de nœud sensibles aux informations de bord

$$\mathbf{f}_i^{\ell}\,\Gamma_{\Theta}(\mathbf{e}_{j,i}^{\mathcal{M}}),$$

dans lequel la couche d'action directe comprend une fonction de réseau neuronal, paramétrée par $\Theta$ ;

le calcul d'une projection scalaire $\mathbf{z_i}$ de la matrice de caractéristiques à valeur réelle $F^{\ell}$ sur un vecteur de projection $\mathbf{v}^{\ell}$, la projection scalaire $\mathbf{z_i}$ comprenant une valeur scalaire projetée de chaque attribut de nœud dans le graphe moléculaire sur le vecteur de projection $\mathbf{v}^{\ell}$, et dans lequel la projection scalaire $\mathbf{z_i}$ mesure en outre des informations de caractéristique du nœud i à retenir lorsqu'elles sont projetées dans la direction du vecteur de projection $\mathbf{v}^{\ell}$ ;

l'obtention d'une propagation hiérarchique par couche d'une couche de regroupement de graphes du graphe moléculaire en prenant un produit des attributs de nœud sensibles aux informations de bord

$$\mathbf{f}_i^{\ell}\,\Gamma_{\Theta}(\mathbf{e}_{j,i}^{\mathcal{M}})$$

et d'un vecteur unitaire associé au vecteur de projection $\mathbf{v}^{\ell}$, dans lequel la direction du vecteur unitaire est la même que la direction du vecteur de projection $\mathbf{v}^{\ell}$;

le sous-échantillonnage du graphe moléculaire en utilisant la propagation hiérarchique par couche de la couche de regroupement de graphes, dans lequel le sous-échantillonnage du graphe moléculaire comprend la réalisation d'une opération de regroupement m-max sur le graphe moléculaire pour échantillonner un sous-ensemble de m nœuds de rang supérieur pour former un graphe moléculaire affiné, dans lequel le sous-échantillonnage du graphe moléculaire a pour résultat le rejet d'un premier ensemble de nœuds et la retenue d'un second ensemble de nœuds parmi une pluralité de nœuds du graphe moléculaire sur la base d'un classement de la pluralité de nœuds, et dans lequel le classement de la pluralité de nœuds est réalisé en utilisant des scores de projection scalaire pour échantillonner des indices du second ensemble de nœuds ;

la détermination d'une première matrice d'adjacence du graphe moléculaire affiné en utilisant le second ensemble de nœuds ; et

la détermination d'une première matrice de caractéristiques du graphe moléculaire affiné en utilisant le second ensemble de nœuds, dans lequel chaque rangée de la première matrice de caractéristiques correspond à des attributs de nœud d'état caché du graphe moléculaire affiné ;

la réalisation d'une ou plusieurs secondes itérations, dans lequel chacune des une ou plusieurs secondes itérations comprend la réalisation, sur le graphe moléculaire affiné d'une itération immédiatement précédente des une ou plusieurs secondes itérations, de l'obtention d'une matrice de caractéristiques à valeur réelle du graphe moléculaire affiné, de la transformation d'attributs de caractéristiques du nœud, du calcul de la projection scalaire $\mathbf{z_i}$ de la matrice de caractéristiques à valeur réelle, de l'obtention de la propagation hiérarchique par couche d'une couche de regroupement de graphes du graphe moléculaire affiné, du sous-échantillonnage du graphe moléculaire affiné en utilisant la propagation hiérarchique par couche de la couche de regroupement de graphes, et de la détermination d'une seconde matrice d'adjacence et d'une seconde matrice de caractéristiques du graphe moléculaire affiné ; et

le calcul d'une moyenne des attributs de nœud d'état caché du graphe moléculaire affiné obtenus après la préformation des une ou plusieurs secondes itérations pour obtenir un vecteur de représentation de niveau de graphe du graphe moléculaire, dans lequel une couche linéaire à partir du vecteur de représentation de niveau de graphe aide à des tâches d'apprentissage inductif pour une prédiction de propriété moléculaire en réduisant la complexité de calcul.

**14.** Un ou plusieurs supports de stockage d'informations lisibles par machine non transitoires selon la revendication 13, dans lequel l'obtention du vecteur de représentation de niveau de graphe du graphe moléculaire comprend :

la réalisation d'une convolution de graphe spatial sur le graphe moléculaire affiné pour transformer et mettre à jour chacun des attributs de nœud d'état caché ;

la réalisation d'un nombre prédéterminé supplémentaire de sous-échantillonnage et de convolution de graphe spatial subséquente sur le graphe moléculaire ;
la réalisation d'un nombre équivalent de sur-échantillonnage et de convolution de graphe spatial subséquente pour rétablir le graphe moléculaire affiné en un clone isomorphe du graphe moléculaire, dans lequel le clone isomorphe du graphe moléculaire est un clone isomorphe transformé par des informations de nœud ; et l'alimentation du clone isomorphe transformé par des informations de nœud du graphe moléculaire en tant qu'entrée à une fonction de lecture invariante d'ordre de nœud pour déterminer le vecteur de représentation de niveau de graphe.

15. Un ou plusieurs supports de stockage d'informations lisibles par machine non transitoires selon la revendication 13, dans lequel l'obtention du vecteur de représentation de niveau de graphe du graphe moléculaire comprend :

la réalisation d'une agrégation de voisinage dynamique spatial sur le graphe moléculaire affiné pour transformer et mettre à jour chacun des attributs de nœud d'état caché ;
la réalisation d'un nombre prédéterminé supplémentaire de sous-échantillonnage et d'agrégation de voisinage dynamique spatial subséquente sur le graphe moléculaire ;
la réalisation d'un nombre équivalent de sur-échantillonnage et d'agrégation de voisinage dynamique spatial subséquente pour rétablir le graphe moléculaire affiné en un clone isomorphe du graphe moléculaire, dans lequel le clone isomorphe du graphe moléculaire est un clone isomorphe transformé par des informations de nœud ; et l'alimentation du clone isomorphe transformé par des informations de nœud du graphe moléculaire en tant qu'entrée à une fonction de lecture invariante d'ordre de nœud pour déterminer le vecteur de représentation de niveau de graphe.

FIG. 1A

FIG. 1B

FIG. 2

ACCESS, VIA ONE OR MORE HARDWARE PROCESSORS, A
DATABASE COMPRISING A PLURALITY OF MOLECULAR GRAPHS
ASSOCIATED WITH A PLURALITY OF MOLECULES AND A
PLURALITY OF LABELS INDICATIVE OF CHEMICAL PROPERTIES OF
THE PLURALITY OF THE MOLECULAR GRAPHS, EACH
MOLECULAR GRAPH OF THE PLURALITY OF MOLECULAR GRAPHS
COMPRISES A PLURALITY OF NODES AND A PLURALITY OF EDGES
CONNECTING THE PLURALITY OF NEIGHBORING NODES
⌐ 302

PERFORMING A FIRST ITERATION TO DOWN-SAMPLE THE
MOLECULAR GRAPH INTO A COARSENED MOLECULAR GRAPH

OBTAIN A REAL-VALUED FEATURE MATRIX OF A
MOLECULAR GRAPH FROM AMONGST THE PLURALITY OF
MOLECULAR GRAPHS, EACH ROW VECTOR OF THE REAL-
VALUED FEATURE MATRIX REPRESENTS A FEATURE
ATTRIBUTE OF THE PLURALITY OF NODES OF THE
MOLECULAR GRAPH
⌐ 306

TRANSFORMING FEATURE ATTRIBUTE $\mathbf{f}_i^\ell$ ASSOCIATED WITH
THE NODE BY TAKING A PRODUCT OF THE FEATURE
ATTRIBUTE $\mathbf{f}_i^\ell$ WITH A FEED-FORWARD LAYER $\Gamma_\Theta(\mathbf{e}_{j,i}^M)$, TO
OBTAIN EDGE-INFORMATION AWARE NODE ATTRIBUTES $\mathbf{f}_i^\ell$
$\Gamma_\Theta(\mathbf{e}_{j,i}^M)$, WHEREIN THE FEED-FORWARD LAYER COMPRISES A
NEURAL NETWORK FUNCTION, PARAMETERIZED BY $\Theta$
⌐ 308

COMPUTING A SCALAR PROJECTION $\mathbf{z}_i$ OF THE REAL-VALUED
FEATURE MATRIX $F^\ell$ ON A PROJECTION VECTOR $\mathbf{v}^\ell$, THE
SCALAR PROJECTION $\mathbf{z}_i$ COMPRISES A PROJECTED SCALAR
VALUE OF EACH NODE-ATTRIBUTE IN THE MOLECULAR
GRAPH ON TO THE PROJECTION VECTOR $\mathbf{v}^\ell$, AND WHEREIN
THE SCALAR PROJECTION $\mathbf{z}_i$ FURTHER MEASURES A FEATURE
INFORMATION OF THE NODE $I$ TO BE RETAINED WHEN
PROJECTED IN THE DIRECTION OF LEARNABLE VECTOR $\mathbf{v}^\ell$
⌐ 310

⌐ 304

OBTAINING A HIERARCHICAL LAYER-WISE PROPAGATION OF
A GRAPH POOLING LAYER OF THE MOLECULAR GRAPH BY
TAKING A PRODUCT OF THE EDGE-INFORMATION AWARE
NODE ATTRIBUTES $\mathbf{f}_i^\ell \Gamma_\Theta(\mathbf{e}_{j,i}^M)$ AND A UNIT VECTOR
ASSOCIATED WITH THE PROJECTION VECTOR $\mathbf{v}^\ell$
⌐ 312

B      A

FIG. 3A

300

B

DOWN-SAMPLE THE MOLECULAR GRAPH USING THE
HIERARCHICAL LAYER-WISE PROPAGATION OF THE GRAPH
POOLING LAYER, WHEREIN THE DOWN-SAMPLING OF THE
MOLECULAR GRAPH COMPRISES PERFORMING A M-MAX-
POOLING OPERATION ON THE MOLECULAR GRAPH TO SAMPLE
A SUBSET OF M TOP-RANKED NODES TO FORM A COARSENED
MOLECULAR GRAPH, WHEREIN THE DOWN-SAMPLING OF THE
MOLECULAR GRAPH RESULTS IN REJECTING A FIRST SET OF
NODES AND RETAINING A SECOND SET OF NODES FROM
AMONGST A PLURALITY OF NODES OF THE MOLECULAR
GRAPH BASED ON A RANKING OF THE PLURALITY OF NODES,
AND WHEREIN THE RANKING OF THE PLURALITY OF NODES IS
PERFORMED BY UTILIZING A SCALAR PROJECTION SCORES TO
SAMPLE INDEXES OF THE SECOND SET OF NODES

314

304

A

DETERMINE A FIRST ADJACENCY MATRIX OF THE COARSENED
MOLECULAR GRAPH USING THE SECOND SET OF NODES

316

DETERMINE A FIRST FEATURE MATRIX OF THE COARSENED
MOLECULAR GRAPH USING THE SECOND SET OF NODES

318

PERFORM ONE OR MORE SECOND ITERATIONS, WHEREIN EACH OF
THE ONE OR MORE SECOND ITERATIONS COMPRISES
PERFORMING, ON THE COARSENED MOLECULAR GRAPH OF AN
IMMEDIATELY PRECEDING ITERATION OF THE ONE OR MORE
SECOND ITERATIONS, OBTAINING A REAL-VALUED FEATURE
MATRIX OF THE COARSENED MOLECULAR GRAPH,
TRANSFORMING FEATURE ATTRIBUTE OF THE NODE, COMPUTING
THE SCALAR PROJECTION $\mathbf{z}_\mathbf{i}$ OF THE REAL-
VALUED FEATURE MATRIX, OBTAINING THE HIERARCHICAL
LAYER-WISE PROPAGATION OF A GRAPH POOLING LAYER OF THE
COARSENED MOLECULAR GRAPH, DOWN-SAMPLING THE
COARSENED MOLECULAR GRAPH USING THE HIERARCHICAL
LAYER-WISE PROPAGATION OF THE GRAPH POOLING LAYER, AND
DETERMINING A SECOND ADJACENCY MATRIX AND A SECOND
FEATURE MATRIX OF THE COARSENED MOLECULAR GRAPH

320

COMPUTE AN AVERAGE OF THE HIDDEN STATE NODE ATTRIBUTES
OF THE COARSENED GRAPH OBTAINED AFTER PREFORMING THE
ONE OR MORE SECOND ITERATIONS TO OBTAIN A GRAPH LEVEL
REPRESENTATION VECTOR OF THE MOLECULAR GRAPH

322

DETERMINE ONE OR MORE MOLECULAR PROPERTIES USING A
LINEAR LAYER FROM THE GRAPH LEVEL REPRESENTATION VECTOR

324

300

FIG. 3B

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121046301

**Non-patent literature cited in the description**

- **GILMER, J. et al.** Neural Message Passing for Quantum Chemistry. *Computer Science*, 04 April 2017, 1-14